(19) Europäisches Patentamt
European Patent Office
Office européen des brevets

(11) EP 2 148 943 B1

(12) **EUROPEAN PATENT SPECIFICATION**

(45) Date of publication and mention of the grant of the patent:
**05.10.2016 Bulletin 2016/40**

(21) Application number: **08747016.7**

(22) Date of filing: **28.04.2008**

(51) Int Cl.:
***C40B 30/04*** *(2006.01)* ***C12Q 1/68*** *(2006.01)*
***C40B 30/08*** *(2006.01)*

(86) International application number:
**PCT/US2008/061760**

(87) International publication number:
**WO 2008/137383 (13.11.2008 Gazette 2008/46)**

(54) **METHODS FOR ASSESSING AND TREATING ULCERATIVE COLITIS AND RELATED DISORDERS USING A 19 GENE PANEL**

METHODEN ZUR UNTERSUCHUNG UND BEHANDLUNG VON COLITIS ULCEROSA UND VERWANDTEN ERKRANKUNGEN UNTER ANWENDUNG EINES PANELS MIT 19 GENEN

PROCEDES POUR EVALUER ET TRAITER LA COLITE ULCERATIVE ET LES TROUBLES ASSOCIES A L'AIDE D'UN PANEL DE 19 GENES

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **30.04.2007 US 914908 P**

(43) Date of publication of application:
**03.02.2010 Bulletin 2010/05**

(73) Proprietor: **Janssen Biotech, Inc.**
**Horsham, PA 19044 (US)**

(72) Inventors:
- **BARIBAUD, Frederic**
  **Radnor, PA 19087 (US)**
- **LI, Xilin**
  **Radnor, PA 19087 (US)**
- **MA, Keying**
  **Radnor, PA 19087 (US)**
- **SONG, Xiao-yu**
  **Somerville, NJ 08876 (US)**

(74) Representative: **Marshall, Cameron John et al**
**Carpmaels & Ransford LLP**
**One Southampton Row**
**London WC1B 5HA (GB)**

(56) References cited:
**WO-A1-2004/001073 WO-A2-2006/063133**
**WO-A2-2006/133287 US-A1- 2004 077 020**

- **SONG XIAO-YU R ET AL: "Characterization of molecular responses to infliximab treatment in ulcerative colitis by expression profiling", GASTROENTEROLOGY; DIGESTIVE DISEASE WEEK MEETING/108TH ANNUAL MEETING OF THE AMERICAN-GASTROENTEROLOGICAL-ASSOCIATION; WASHINGTON, DC, USA; MAY 19 -24, 2007, ELSEVIER, PHILADELPHIA, PA, vol. 132, no. 4 supplement 2, 1 April 2007 (2007-04-01), page A145, XP008132767, ISSN: 0016-5085**
- **'Information for probe set 1557488_at (HG-U133_Plus_2)', [Online] 18 September 2013, GENE ANNOT, page 1, XP055079914 Retrieved from the Internet: <URL:http://genecards.weizmann.ac.il/cgi-bin/geneannot/GA_search.pl?keyword_type=probe _set_id&array=HG-U133_Plus_2&target=genecar ds&keyword=1557488_at> [retrieved on 2013-09-18]**
- **GREENBAUM DOV ET AL: "Interrelating different types of genomic data, from proteome to secretome: 'Oming in on function", GENOME RESEARCH, COLD SPRING HARBOR LABORATORY PRESS, WOODBURY, NY, US, vol. 11, no. 9, 1 September 2001 (2001-09-01), pages 1463-1468, XP009088703, ISSN: 1088-9051, DOI: 10.1101/GR.207401**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## FIELD OF THE INVENTION

The invention relates to the identification of expression profiles and the nucleic acids indicative of gastrointestinal-related disorders, such as ulcerative colitis, and to the use of such expression profiles and nucleic acids in diagnosis of ulcerative colitis and related diseases. The invention further relates to methods for identifying, using, and testing candidate agents and/or targets which modulate ulcerative colitis.

## BACKGROUND OF THE INVENTION

Ulcerative colitis (UC) is a multifactorial autoimmune disease with a complex pathogenesis involving unidentified genetic, microbial, and environmental factors. Recent studies using microarray analysis of inflamed colonoscopic tissue biopsy vs. non-inflamed biopsy samples from UC patients revealed dysregulation of a few inflammatory cytokines (1), however, the etiology, pathogenesis, and role of tumor necrosis factor-alpha (TNF$\alpha$) in UC is still poorly understood. TNF$\alpha$ is a critical proinflammatory cytokine in Crohn's disease as demonstrated by the therapeutic effect of infliximab on the induction and maintenance of clinical remission, closure of enterocutaneous, perianal, and rectovaginal fistulas, maintenance of fistula closure, and steroid tapering in Crohn's disease patients (2-5). However, the evidence to support a role of TNF$\alpha$ in the pathogenesis of UC has been controversial (6-10) despite the fact that it is also found at increased levels in the blood, colonic tissue, and stools of UC patients (11-13). A recent clinical study (ACT-1) by Rutgeerts et al. showed that infliximab is effective when administered at weeks 0, 2, 6 and every 8 weeks thereafter in achieving clinical response and remission in patients with moderate-to-severe active UC despite the use of conventional therapy supporting a critical pathogenic role of TNF$\alpha$ in UC.

Microarray technology is a powerful tool since it enables analysis of the expression of thousands of genes simultaneously and can also be automated allowing for a high-throughput format. In diseases associated with complex host functions, such as those known as immune mediated inflammatory diseases, such as UC, microarray results can provide a gene expression profile that can be of utility in designing new approaches to disease diagnosis and management. These approaches also serve to identify novel genes and annotating genes of unknown function heretofore unassociated with the disease or condition. Accordingly, there is a need to identify and characterize new gene markers useful in developing methods for diagnosing and treating autoimmune disorders, such as UC and Crohn's disease, as well as other diseases and conditions and how a patient would respond to a therapeutic intervention.

Gene expression can be modulated in several different ways, including by the use of siRNAs, shRNAs, antisense molecules and DNAzymes. SiRNAs and shRNAs both work via the RNAi pathway and have been successfully used to suppress the expression of genes. RNAi was first discovered in worms and the phenomenon of gene silencing related to dsRNA was first reported in plants by Fire and Mello and is thought to be a way for plant cells to combat infection with RNA viruses. In this pathway, the long dsRNA viral product is processed into smaller fragments of 21-25 bp in length by a DICER-like enzyme and then the double-stranded molecule is unwound and loaded into the RNA induced silencing complex (RISC). A similar pathway has been identified in mammalian cells with the notable difference that the dsRNA molecules must be smaller than 30 bp in length in order to avoid the induction of the so-called interferon response, which is not gene specific and leads to the global shut down of protein synthesis in the cell.

Synthetic siRNAs have been successfully designed to selectively target a single gene and can be delivered to cells in vitro or in vivo. ShRNAs are the DNA equivalents of siRNA molecules and have the advantage of being incorporated into a cells' genome where they are replicated during every mitotic cycle.

DNAzymes have also been used to modulate gene expression. DNAzymes are catalytic DNA molecules that cleave single-stranded RNA. They are highly selective for the target RNA sequence and as such can be used to down-regulate specific genes through targeting of the messenger RNA.

Accordingly, there is a need to identify and characterize new gene markers useful in developing methods for diagnosing and treating autoimmune disorders, such as UC and Crohn's disease, as well as other diseases and conditions.

## Summary of the Invention

The present invention includes the discovery of panels of genes, one of 19 genes and one of five genes, that have modified expression levels in patients responsive to treatment for UC (effective in reducing the symptoms of UC) versus patients nonresponsive to treatment. The modified expression levels constitute a profile that can serve as a biomarker profile predictive of a patient's responsiveness to treatment.

In a particular embodiment, the present invention comprises a method of predicting the suitability of a treatment for UC based on the pattern of gene expression of one or more of the 19 genes which constitute the profile prior to treatment, as disclosed in claim 1. One or more of these genes may be from a category of genes, such as those involved in nucleotide acid binding, ATP binding, transferase activity, proteolysis, oxidoreductase activity, ubiquitin thiolesterase activity, replication of proteins, signal transduction, and regulation of transcription, and the like. In a typical embodiment, the cell specimen expresses at least two expression profile genes. The profile genes may show an increase or decrease.

In addition, the present invention comprises a method of identifying subjects with UC and/or related diseases or disorders that are candidates for treatment with a particular therapeutic agent by evaluating their expression profile of one or more genes of the 19- or 5-gene panel.

In one embodiment, the UC-related gene profile is used to create an array-based method for prognostic or diagnostic purposes, the method comprising:

(a) preparing a representative mixture of nucleic acids from a specimen obtained from a patient and causing said sample nucleic acids in the mixture to be labeled with a detectable marker;

(b) contacting a sample with an array comprising a plurality of nucleic acid segments, wherein each nucleic acid segment is immobilized to a discrete and known address on a substrate surface wherein the panel of UC-related biomarkers is identified as a feature of the array by address, the array further comprises at least one calibration nucleic acid at a known address on the substrate, and contacting is performed under conditions in which a sample nucleic acid specifically may bind to the nucleic acid segment immobilized on the arrays;

(c) performing a statistical comparison of all test samples from treated patients and a reference standard; and

(d) comparing the pattern of intensity changes in features for the test sample to the pattern of intensity changes for those features which are members of the UC-related gene profile with historical patterns for samples taken from patients responsive to treatment with an anti-TNF antibody.

Optionally, statistical analysis is performed on the changes in levels of members of the gene panel to evaluate the significance of these changes and to identify which members are meaningful members of the panel. Also described is a kit for predicting the suitability of candidate agents for treating UC and/or related diseases or disorders based on the pattern of gene expression.

**Detailed Description of the Invention**

Definitions

The following definitions are set forth to illustrate and define the meaning and scope of various terms used to describe the invention herein.

An "activity," a biological activity, and a functional activity of a polypeptide refers to an activity exerted by a gene of the UC-related gene panel in response to its specific interaction with another protein or molecule as determined in vivo, in situ, or in vitro, according to standard techniques. Such activities can be a direct activity, such as an association with or an enzymatic activity on a second protein, or an indirect activity, such as a cellular process mediated by interaction of the protein with a second protein or a series of interactions as in intracellular signaling or the coagulation cascade.

An "antibody" includes any polypeptide or peptide containing molecule that comprises at least a portion of an immunoglobulin molecule, such as but not limited to, at least one complementarity determining region (CDR) of a heavy or light chain or a ligand binding portion thereof, a heavy chain or light chain variable region, a heavy chain or light chain constant region, a framework region, or any portion, fragment or variant thereof. The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. For example, antibody fragments include, but are not limited to, Fab (e.g., by papain digestion), Fab' (e.g., by pepsin digestion and partial reduction) and F(ab')2 (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, and single domain antibodies (e.g., $V_H$ or $V_L$), are encompassed by the invention (see, e.g., Colligan, et al., eds., Current Protocols in Immunology, John Wiley & Sons, Inc., NY (1994-2001); Colligan et al., Current Protocols in Polypeptide Science, John Wiley & Sons, NY (1997-2001)).

The terms "array" or "microarray" or "biochip" or "chip" as used herein refer to articles of manufacture or devices comprising a plurality of immobilized target elements, each target element comprising a "clone," "feature," "spot" or defined area comprising a particular composition, such as a biological molecule, e.g., a nucleic acid molecule or polypeptide, immobilized to a solid surface, as discussed in further detail, below.

"Complement of" or "complementary to" a nucleic acid sequence of the invention refers to a polynucleotide molecule having a complementary base sequence and reverse orientation as compared to a first polynucleotide.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" and "similarity" can be readily calculated by known methods, including, but not limited to, those described in Computational Molecular Biology, Lesk, A. M., ed., Oxford University Press, New York, 1988; Biocomputing:Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., Siam J. Applied Math., 48:1073 (1988). In addition, values for percentage identity can be obtained from amino acid and nucleotide sequence alignments generated using the default settings for the AlignX component of Vector NTI Suite 8.0 (Informax, Frederick, MD).

The terms "specifically hybridize to," "hybridizing specifically to," "specific hybridization" and "selectively hybridize to," as used herein refer to the binding, duplexing, or hybridizing of a nucleic acid molecule preferentially to a particular nucleotide sequence under stringent conditions. The term "stringent conditions" refers to conditions under which a probe will hybridize preferentially to its target subsequence; and to a lesser extent to, or not at all to, other sequences. A "stringent hybridization" and "stringent hybridization wash conditions" in the context of nucleic acid hybridization (e.g., as in array, Southern or Northern hybridizations) are sequence dependent, and are different under different environmental parameters. Alternative hybridization conditions that can be used to practice the invention are described in detail, below. In alternative aspects, the hybridization and/or wash conditions are carried out under moderate conditions, stringent conditions and very stringent conditions, as described in further detail, below. Alternative wash conditions are also used in different aspects, as described in further detail, herein.

The phrases "labeled biological molecule" or "labeled with a detectable composition" or "labeled with a detectable moiety" as used herein refer to a biological molecule, e.g., a nucleic acid, comprising a detectable composition, i.e., a label, as described in detail, below. The label can also be another biological molecule, as a nucleic acid, e.g., a nucleic acid in the form of a stem-loop structure as a "molecular beacon," as described below. This includes incorporation of labeled bases (or, bases which can bind to a detectable label) into the nucleic acid by, e.g., nick translation, random primer extension, amplification with degenerate primers, and the like. Any label can be used, e.g., chemiluminescent labels, radiolabels, enzymatic labels and the like. The label can be detectable by any means, e.g., visual, spectroscopic, photochemical, biochemical, immunochemical, physical, chemical and/or chemiluminescent detection. The invention can use arrays comprising immobilized nucleic acids comprising detectable labels.

The term "nucleic acid" as used herein refers to a deoxyribonucleotide (DNA) or ribonucleotide (RNA) in either single- or double-stranded form. The term encompasses nucleic acids containing known analogues of natural nucleotides. The term nucleic acid is used interchangeably with gene, DNA, RNA, cDNA, mRNA, oligonucleotide primer, probe and amplification product. The term also encompasses DNA backbone analogues, such as phosphodiester, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs).

The terms "sample" or "sample of nucleic acids" as used herein refer to a sample comprising a DNA or RNA, or nucleic acid representative of DNA or RNA isolated from a natural source. A "sample of nucleic acids" is in a form suitable for hybridization (e.g., as a soluble aqueous solution) to another nucleic acid (e.g., immobilized probes). The sample nucleic acid may be isolated, cloned, or extracted from particular cells or tissues. The cell or tissue sample from which the nucleic acid sample is prepared is typically taken from a patient having or suspected of having UC or a related disease or condition. Methods of isolating cell and tissue samples are well known to those of skill in the art and include, but are not limited to, aspirations, tissue sections, needle biopsies, and the like. Frequently the sample will be a "clinical sample" which is a sample derived from a patient, including sections of tissues such as frozen sections or paraffin sections taken for histological purposes. The sample can also be derived from supernatants (of cells) or the cells themselves taken from patients or from cell cultures, cells from tissue culture and other media in which it may be desirable to detect the response to drug candidates. In some cases, the nucleic acids may be amplified using standard techniques such as PCR, prior to the hybridization. The probe an be produced from and collectively can be representative of a source of nucleic acids from one or more particular (pre-selected) portions of, e.g., a collection of polymerase chain reaction (PCR) amplification products, substantially an entire chromosome or a chromosome fragment, or substantially an entire genome, e.g., as a collection of clones, e.g., BACs, PACs, YACs, and the like (see below).

"Nucleic acids" are polymers of nucleotides, wherein a nucleotide comprises a base linked to a sugar which sugars are in turn linked one to another by an interceding at least bivalent molecule, such as phosphoric acid. In naturally occurring nucleic acids, the sugar is either 2'-deoxyribose (DNA) or ribose (RNA). Unnatural poly- or oliogonucleotides contain modified bases, sugars, or linking molecules, but are generally understood to mimic the complementary nature of the naturally occurring nucleic acids after which they are designed. An example of an unnatural oligonucleotide is an antisense molecule composition that has a phosphorothiorate backbone. An "oligonucleotide" generally refers to a nucleic acid molecule having less than 30 nucleotides.

The term "profile" means a pattern and relates to the magnitude and direction of change of a number of features. The profile may be interpreted stringently, i.e., where the variation in the magnitude and/or number of features within the profile displaying the characteristic is substantially similar to a reference profile or it may be interpreted less stringently, for example, by requiring a trend rather than an absolute match of all or a subset of feature characteristics.

The terms "protein," "polypeptide," and "peptide" include "analogs," or "conservative variants" and "mimetics" or "peptidomimetics" with structures and activity that substantially correspond to the polypeptide from which the variant was derived, as discussed in detail above.

A "polypeptide" is a polymer of amino acid residues joined by peptide bonds, and a peptide generally refers to amino acid polymers of 12 or less residues. Peptide bonds can be produced naturally as directed by the nucleic acid template or synthetically by methods well known in the art.

A "protein" is a macromolecule comprising one or more polypeptide chains. A protein may further comprise substituent groups attached to the side groups of the amino acids not involved in formation of the peptide bonds. Typically, proteins formed by eukaryotic cell expression also contain carbohydrates. Proteins are defined herein in terms of their amino acid sequence or backbone and substituents are not specified, whether known or not.

The term "receptor" denotes a molecule having the ability to affect biological activity, in e.g., a cell, as a result of interaction with a specific ligand or binding partner. Cell membrane bound receptors are characterized by an extracellular ligand-binding domain, one or more membrane spanning or transmembrane domains, and an intracellular effector domain that is typically involved in signal transduction. Ligand binding to cell membrane receptors causes changes in the extracellular domain that are communicated across the cell membrane, direct or indirect interaction with one or more intracellular proteins, and alters cellular properties, such as enzyme activity, cell shape, or gene expression profile. Receptors may also be untethered to the cell surface and may be cytosolic, nuclear, or released from the cell altogether. Non-cell associated receptors are termed soluble receptors or ligands.

Publications refer to any scientific or patent publications, or any other information available in any media format, including all recorded, electronic or printed formats.

## Gene Panel Identification and Validation

Also described are methods for screening for compositions which modulate the symptoms of UC, particularly the mucosal layer of the rectum and all or part of the colon. By "UC" or grammatical equivalents as used herein, is meant a disease state or condition which is marked by diarrhea, rectal bleeding, tenesmus, passage of mucus, and crampy abdominal pain.

In one aspect, the expression levels of genes are determined in different patient samples for which diagnosis information is desired, to provide expression profiles. An expression profile of a particular sample is essentially a "fingerprint" of the state of the sample; while two states may have any particular gene similarly expressed, the evaluation of a number of genes simultaneously allows the generation of a gene expression profile that is unique to the state of the patient sample. That is, normal tissue may be distinguished from lesion tissue and tissue from a treated patient may be distinguished from an untreated patient. By comparing expression profiles of tissue in different disease states that are known, information regarding which genes are important (including both up- and down-regulation of genes) in each of these states is obtained.

The identification of sequences (genes) that are differentially expressed in disease tissue allows the use of this information in a number of ways. For example, the evaluation of a particular treatment regime may be evaluated.

This may be done by making biochips comprising sets of the important disease genes, which can then be used in these screens. These methods can also be performed on the protein basis; that is, protein expression levels of the UC-related gene product proteins can be evaluated for diagnostic purposes or to screen candidate agents. In addition, the nucleic acid sequences comprising the UC-related gene profile can be used to measure whether a patient is likely to respond to a therapeutic prior to treatment.

UC-related gene sequences can include both nucleic acid and amino acid sequences. In a preferred embodiment, the UC-related gene sequences are recombinant nucleic acids. By the term "recombinant nucleic acid" herein is meant nucleic acid, originally formed in vitro, in general, by the manipulation of nucleic acid by polymerases and endonucleases, in a form not normally found in nature. Thus, an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e., using the in vivo cellular machinery of the host cell rather than in vitro manipulations; however, such nucleic acids, once produced recombinantly, although subsequently replicated non-recombinantly, are still considered recombinant for the purposes of the invention.

**Method of Practicing the Invention**

The invention provides in silico, array-based methods relying on the relative amount of a binding molecule (e.g., nucleic acid sequence) in two or more samples. Also provided are computer- implemented methods for determining the relative amount of a binding molecule (e.g., nucleic acid sequence) in two or more samples and using the determined relative binding amount to predict responsiveness to a particular therapy, and monitor and enhance therapeutic treatment.

In practicing the methods of the invention, two or more samples of labeled biological molecules (e.g., nucleic acid) are applied to two or more arrays, where the arrays have substantially the same complement of immobilized binding molecule (e.g., immobilized nucleic acid capable of hybridizing to labeled sample nucleic acid). The two or more arrays are typically multiple copies of the same array. However, because each "spot," "clone" or "feature" on the array has similar biological molecules (e.g., nucleic acids of the same sequence) and the biological molecules (e.g., nucleic acid) in each spot is known, as is typical of nucleic acid and other arrays, it is not necessary that the multiple arrays used in the invention be identical in configuration it is only necessary that the position of each feature on the substrate be known, that is, have an address. Thus, in one aspect, multiple biological molecules (e.g., nucleic acid) in samples are comparatively bound to the array (e.g., hybridized simultaneously) and the information gathered is coded so that the results are based on the inherent properties of the feature (e.g., the nucleic acid sequence) and not it's position on the substrate.

Amplification of Nucleic Acids

Amplification using oligonucleotide primers can be used to generate nucleic acids used in the compositions and methods of the invention, to detect or measure levels of test or control samples hybridized to an array, and the like. The skilled artisan can select and design suitable oligonucleotide amplification primers. Amplification methods are also well known in the art, and include, e.g., polymerase chain reaction, PCR (PCR PROTOCOLS, A GUIDE TO METHODS AND APPLICATIONS, ed. Innis, Academic Press, N.Y. (1990) and PCR STRATEGIES (1995), ed. Innis, Academic Press, Inc., N.Y., ligase chain reaction (LCR) (see, e.g., Wu (1989) Genomics 4:560; Landegren (1988) Science 241:1077; Barringer (1990) Gene 89:117); transcription amplification (see, e.g., Kwoh (1989) Proc. Natl. Acad. Sci. USA 86:1173); and, self-sustained sequence replication (see, e.g., Guatelli (1990) Proc. Natl. Acad. Sci. USA 87:1874); Q Beta replicase amplification (see, e.g., Smith (1997) J. Clin. Microbiol. 35:1477-1491), automated Q-beta replicase amplification assay (see, e.g., Burg (1996) Mol. Cell. Probes 10:257-271) and other RNA polymerase mediated techniques (e.g., NASBA, Cangene, Mississauga, Ontario); see also Berger (1987) Methods Enzymol. 152:307-316; Sambrook; Ausubel; U.S. Pat. Nos. 4,683,195 and 4,683,202; Sooknanan (1995) Biotechnology 13:563-564.

Hybridizing Nucleic Acids

In practicing the methods of the invention, test and control samples of nucleic acid are hybridized to immobilized probe nucleic acid, e.g., on arrays. In alternative aspects, the hybridization and/or wash conditions are carried out under moderate conditions, stringent conditions and very stringent conditions. An extensive guide to the hybridization of nucleic acids is found in, e.g., Sambrook Ausubel, Tijssen. Generally, highly stringent hybridization and wash conditions are selected to be about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Very stringent conditions are selected to be equal to the Tm for a particular probe. An example of stringent hybridization conditions for hybridization of complementary nucleic acids which have more than 100 complementary residues on an array or a filter in a Southern or northern blot is 42°C using standard hybridization solutions (see, e.g., Sambrook), with the hybridization being carried out overnight. An example of highly stringent wash conditions is 0.15 M NaCl at 72°C for about 15 minutes. An example of stringent wash conditions is a 0.2×SSC wash at 65°C for 15 minutes (see, e.g., Sambrook). Often, a high stringency wash is preceded by a medium or low stringency wash to remove background probe signal. An example medium stringency wash for a duplex of, e.g., more than 100 nucleotides, is 1×SSC at 45°C for 15 minutes. An example of a low stringency wash for a duplex of, e.g., more than 100 nucleotides, is 4× to 6×SSC at 40° C for 15 minutes.

In alternative aspects of the compositions and methods of the invention, e.g., in practicing comparative nucleic acid hybridization, such as comparative genomic hybridization (CGH) with arrays, the fluorescent dyes Cy3® and Cy5® are used to differentially label nucleic acid fragments from two samples, e.g., the array-immobilized nucleic acid versus the sample nucleic acid, or, nucleic acid generated from a control versus a test cell or tissue. Many commercial instruments are designed to accommodate the detection of these two dyes. To increase the stability of Cy5®, or fluors or other oxidation-sensitive compounds, antioxidants and free radical scavengers can be used in hybridization mixes, the hybridization and/or the wash solutions. Thus, Cy5® signals are dramatically increased and longer hybridization times are possible. See WO 0194630 A2 and U.S. Patent Application No. 20020006622.

To further increase the hybridization sensitivity, hybridization can be carried out in a controlled, unsaturated humidity environment; thus, hybridization efficiency is significantly improved if the humidity is not saturated. See WO 0194630 A2 and U.S. Patent Application No. 20020006622. The hybridization efficiency can be improved if the humidity is dynamically controlled, i.e., if the humidity changes during hybridization. Mass transfer will be facilitated in a dynamically balanced humidity environment. The humidity in the hybridization environment can be adjusted stepwise or continuously. Array devices comprising housings and controls that allow the operator to control the humidity during pre-hybridization, hybridization, wash and/or detection stages can be used. The device can have detection, control and memory components to allow pre-programming of the humidity and temperature controls (which are constant and precise or which flucturate), and other parameters during the entire procedural cycle, including pre-hybridization, hybridization, wash and detection steps. See WO 0194630 A2 and U.S. Patent Application No. 20020006622.

The methods of the invention can comprise hybridization conditions comprising osmotic fluctuation. Hybridization efficiency (i.e., time to equilibrium) can also be enhanced by a hybridization environment that comprises changing hyper-/hypo-tonicity, e.g., a solute gradient. A solute gradient is created in the device. For example, a low salt hybridization solution is placed on one side of the array hybridization chamber and a higher salt buffer is placed on the other side to generate a solute gradient in the chamber. See WO 0194630 A2 and U.S. Patent Application No. 20020006622.

<u>Blocking the Ability of Repetitive Nucleic Acid Sequences to Hybridize</u>

The methods of the invention can comprise a step of blocking the ability of repetitive nucleic acid sequences to hybridize (i.e., blocking "hybridization capacity") in the immobilized nucleic acid segments. The hybridization capacity of repetitive nucleic acid sequences in the sample nucleic acid sequences can be blocked by mixing sample nucleic acid sequences with unlabeled or alternatively labeled repetitive nucleic acid sequences. Sample nucleic acid sequences can be mixed with repetitive nucleic acid sequences before the step of contacting with the array-immobilized nucleic acid segments. Blocking sequences are for example, Cot-1 DNA, salmon sperm DNA, or specifc repetitive genomic sequences. The repetitive nucleic acid sequences can be unlabeled. A number of methods for removing and/or disabling the hybridization capacity of repetitive sequences using, e.g., Cot-1 are known; see, e.g., Craig (1997) Hum. Genet. 100:472-476; WO 93/18186. Repetitive DNA sequences can be removed from library probes by means of magnetic purification and affinity PCR, see, e.g., Rauch (2000) J. Biochem. Biophys. Methods 44:59-72.

Arrays are generically a plurality of target elements immobilized onto the surface of the plate as defined "spots" or "clusters," or "features," with each target element comprising one or more biological molecules (e.g., nucleic acids or polypeptides) immobilized to a solid surface for specific binding (e.g., hybridization) to a molecule in a sample. The immobilized nucleic acids can contain sequences from specific messages (e.g., as cDNA libraries) or genes (e.g., genomic libraries), including a human genome. Other target elements can contain reference sequences and the like. The biological molecules of the arrays may be arranged on the solid surface at different sizes and different densities. The densities of the biological molecules in a cluster and the number of clusters on the array will depend upon a number of factors, such as the nature of the label, the solid support, the degree of hydrophobicity of the substrate surface, and the like. Each feature may comprise substantially the same biological molecule (e.g., nucleic acid), or, a mixture of biological molecules (e.g., nucleic acids of different lengths and/or sequences). Thus, for example, a feature may contain more than one copy of a cloned piece of DNA, and each copy may be broken into fragments of different lengths.

Array substrate surfaces onto which biological molecules (e.g., nucleic acids) are immobilized can include nitrocellulose, glass, quartz, fused silica, plastics and the like, as discussed further, below. The compositions and methods of the invention can incorporate in whole or in part designs of arrays, and associated components and methods, as described, e.g., in U.S. Pat. Nos. 6,344,316; 6,197,503; 6,174,684; 6,159,685; 6,156,501; 6,093,370; 6,087,112; 6,087, 103; 6,087,102; 6,083,697; 6, 080,585; 6,054,270; 6,048,695; 6,045,996; 6,022,963; 6,013,440; 5,959,098; 5,856,174; 5,843,655; 5,837,832; 5,770,456; 5,723,320; 5,700,637; 5,695, 940; 5,556,752; 5,143,854; see also, e.g., WO 99/51773; WO 99/09217; WO 97/46313; WO 96/17958; WO 89/10977; see also, e.g., Johnston (1998) Curr. Biol. 8:R171-174; Schummer (1997) Biotechniques 23:1087-1092; Kern (1997) Biotechniques 23:120-124; Solinas- Toldo (1997) Genes, Chromosomes & Cancer 20:399-407; Bowtell (1999) Nature Genetics Supp. 21:25-32; Epstein (2000) Current Opinion in Biotech. 11:36-41; Mendoza (1999 Biotechniques 27: 778-788; Lueking (1999) Anal. Biochem. 270:103-111; Davies (1999) Biotechniques 27:1258-1261.

<u>Substrate Surfaces</u>

Substrate surfaces that can be used in the compositions and methods of the invention include, for example, glass (see, e.g., U.S. Pat. No. 5,843,767), ceramics, and quartz. The arrays can have substrate surfaces of a rigid, semi-rigid or flexible material. The substrate surface can be flat or planar, be shaped as wells, raised regions, etched trenches, pores, beads, filaments, or the like. Substrate surfaces can also comprise various materials such as nitrocellulose, paper, crystalline substrates (e.g., gallium arsenide), metals, metalloids, polacryloylmorpholide, various plastics and plastic copolymers, Nylon®, Teflon®, polyethylene, polypropylene, latex, polymethacrylate, poly (ethylene terephthalate), rayon, nylon, poly(vinyl butyrate), and cellulose acetate. The substrates may be coated and the substate and the coating may be functionalized to, e.g., enable conjugation to an amine.

Arrays Comprising Calibration Sequences

The invention comtemplates the use of arrays comprising immobilized calibration sequences for normalizing the results of array-based hybridization reactions, and methods for using these calibration sequences, e.g., to determine the copy number of a calibration sequence to "normalize" or "calibrate" ratio profiles. The calibration sequences can be substantially the same as a unique sequence in an immobilized nucleic acid sequence on an array. For example, a "marker" sequence from each "spot" or "biosite" on an array (which is present only on that spot, making it a "marker" for that spot) is represented by a corresponding sequence on one or more "control" or "calibration" spot(s).

The "control spots" or "calibration spots" are used for "normalization" to provide information that is reliable and repeatable. Control spots can provide a consistent result independent of the labeled sample hybridized to the array (or a labeled binding molecule from a sample). The control spots can be used to generate a "normalization" or "calibration" curve to offset possible intensity errors between the two arrays (or more) used in the in silico, array-based methods of the invention.

One method of generating a control on the array would be to use an equimolar mixture of all the biological molecules (e.g., nucleic acid sequences) spotted on the array and generating a single spot. This single spot would have equal amounts of the biological molecules (e.g., nucleic acid sequences) from all the other spots on the array. Multiple control spots can be generated by varying the concentration of the equimolar mixture.

**Samples and Specimens**

The sample nucleic acid may be isolated, cloned, or extracted from particular cells, tissues, or other specimens. The cell or tissue sample from which the nucleic acid sample is prepared is typically taken from a patient having or suspected of having UC or a related condition. Methods of isolating cell and tissue samples are well known to those of skill in the art and include, but are not limited to, aspirations, tissue sections, needle biopsies, and the like. Frequently, the sample will be a "clinical sample" which is a sample derived from a patient, including whole blood, or sections of tissues, such as frozen sections or paraffin sections taken for histological purposes. The sample can also be derived from supernatants (of cells) or the cells themselves taken from patients or from cell cultures, cells from tissue culture and other media in which it may be desirable to detect the response to drug candidates. In some cases, the nucleic acids may be amplified using standard techniques such as PCR, prior to the hybridization.

Also described is a pre-treatment method of predicting disease regression or resolution. The method includes (1) taking a colon biopsy or other specimen from an individual diagnosed with UC or a related disease or disorder, (2) measuring the expression levels of the profile genes of the panel, (3) comparing the pre-treatment expression level of the genes with a pre-treatment reference profile from treatment responders, and (4) predicting treatment response by monitoring the expression levels of the gene panel.

**Methods of Assessing Biomarker Utililty**

The prognostic utility of the present biomarker gene panel for assessing a patient's response to treatment or prognosis of disease can be validated by using other means for assessing a patient's state of disease. For example, gross measurement of disease may be assessed and recorded by certain imaging methods, such as but not limited to: imaging by photographic, radiometric, or magnetic resonance technology. General indices of health or disease futher include serum or blood composition (protein, liver enzymes, pH, electrolytes, red cell volume, hematocrit, hemoglobin, or specific protein). However, in some diseases, the etiology is still poorly understood. UC is an example of one such disease.

Patient Assessment and Monitoring

Some of the genes in the panel belong to classes of genes that have been reported to be aberrantly expressed in UC patients previously, such as transcription factors, replication proteins, and oxidases, the expression patterns of the genes over the course of treatment have not been studied in the treatment of UC, and none has been identified as having predictive value. The panel of gene expression biomarkers disclosed herein permits the generation of methods for rapid and reliable prediction, diagnostic tools that predict the clinical outcome of a UC trial, or prognostic tools for tracking the efficacy of UC therapy. Prognostic methods based on detecting these genes in a sample are provided. These compositions may be used, for example, in connection with the diagnosis, prevention and treatment of a range of immune-mediated inflammatory diseases.

Therapeutic agents

Antagonists

As used herein, the term "antagonists" refer to substances which inhibit or neutralize the biologic activity of the gene product of the UC-related gene panel of the invention. Such antagonists accomplish this effect in a variety of ways. One class of antagonists will bind to the gene product protein with sufficient affinity and specificity to neutralize the biologic effects of the protein. Included in this class of molecules are antibodies and antibody fragments (such as, for example, F(ab) or $F(ab')_2$ molecules). Another class of antagonists comprises fragments of the gene product protein, muteins or small organic molecules, i.e., peptidomimetics, that will bind to the cognate binding partners or ligands of the gene product, thereby inhibiting the biologic activity of the specific interaction of the gene product with its cognate ligand or receptor. The UC-related gene antagonist may be of any of these classes as long as it is a substance that inhibits at least one biological activity of the gene product.

Antagonists include antibodies directed to one or more regions of the gene product protein or fragments thereof, antibodies directed to the cognate ligand or receptor, and partial peptides of the gene product or its cognate ligand which inhibit at least one biological activity of the gene product. Another class of antagonists includes siRNAs, shRNAs, antisense molecules and DNAzymes targeting the gene sequence as known in the art are disclosed herein.

Suitable antibodies include those that compete for binding to UC-related gene products with monoclonal antibodies that block UC-related gene product activation or prevent UC-related gene product binding to its cognate ligand, or prevent UC-related gene product signalling.

A therapeutic targeting the inducer of the UC-related gene product may provide better chances of success. Gene expression can be modulated in several different ways including by the use of siRNAs, shRNAs, antisense molecules and DNAzymes. Synthetic siRNAs, shRNAs, and DNAzymes can be designed to specifically target one or more genes and they can easily be delivered to cells in vitro or in vivo.

Also described are antisense nucleic acid molecules, i.e., molecules that are complementary to a sense nucleic acid encoding a UC-related gene product polypeptide, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire coding strand, or to only a portion thereof, e.g., all or part of the protein coding region (or open reading frame). An antisense nucleic acid molecule can be antisense to all or part of a non-coding region of the coding strand of a nucleotide sequence encoding a UC-related gene product polypeptide. The non-coding regions ("5' and 3' untranslated regions") are the 5' and 3' sequences that flank the coding region and are not translated into amino acids.

Also described are chimeric or fusion proteins. As used herein, a "chimeric protein" or "fusion protein" comprises all or part (preferably biologically active) of a UC-related gene product polypeptide operably linked to a heterologous polypeptide (i.e., a polypeptide other than the same UC-related gene product polypeptide). Within the fusion protein, the term "operably linked" is intended to indicate that the UC-related gene product polypeptide and the heterologous polypeptide are fused in-frame to each other. The heterologous polypeptide can be fused to the amino-terminus or the carboxyl-terminus of the UC-related gene product polypeptide. In another embodiment, a UC-related gene product polypeptide or a domain or active fragment thereof can be fused with a heterologous protein sequence or fragment thereof to form a chimeric protein, where the polypeptides, domains or fragments are not fused end to end but are interposed within the heterologous protein framework.

In yet another embodiment, the fusion protein is an immunoglobulin fusion protein in which all or part of a UC-related gene product polypeptide is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a UC-related gene product polypeptide. Inhibition of ligand/receptor interaction can be useful therapeutically, both for treating proliferative and differentiative disorders and for modulating (e.g., promoting or inhibiting) cell survival. A preferred embodiment of an immunoglobulin chimeric protein is a $C_H1$ domain-deleted immunoglobulin or MIMETIBODY™ construct having an active polypeptide fragment interposed within a modified framework region as taught in copending application PCT WO/04002417. Moreover, the immunoglobulin fusion proteins of the invention can be used as immunogens to produce antibodies directed against a UC-related gene product polypeptide in a subject, to purify ligands and in screening assays to identify molecules that inhibit the interaction of receptors with ligands.

Compositions and Their Uses

In accordance with the invention, the neutralizing anti-UC-related gene product antagonists, such as monoclonal antibodies, described herein can be used to inhibit UC-related gene product activity. Additionally, such antagonists can be used to inhibit the pathogenesis of UC and -related inflammatory diseases amenable to such treatment, which may include, but are not limited to, rheumatic diseases. The individual to be treated may be any mammal and is preferably a primate, a companion animal which is a mammal and most preferably a human patient. The amount of antagonist administered will vary according to the purpose it is being used for and the method of administration.

The UC-related gene antagonists may be administered by any number of methods that result in an effect in tissue in which pathological activity is desired to be prevented or halted. Further, the anti-UC-related gene product antagonists need not be present locally to impart an effect on the UC-related gene product activity, therefore, they may be administered wherever access to body compartments or fluids containing UC-related gene product is achieved. In the case of inflamed, malignant, or otherwise compromised tissues, these methods may include direct application of a formulation containing the antagonists. Such methods include intravenous administration of a liquid composition, transdermal administration of a liquid or solid formulation, oral, topical administration, or interstitial or inter-operative administration. Adminstration may be affected by the implantation of a device whose primary function may not be as a drug delivery vehicle.

For antibodies, the preferred dosage is about 0.1 mg/kg to 100 mg/kg of body weight (generally about 10 mg/kg to 20 mg/kg). If the antibody is to act in the brain, a dosage of about 50 mg/kg to 100 mg/kg is usually appropriate. Generally, partially human antibodies and fully human antibodies have a longer half-life within the human body than other antibodies. Accordingly, the use of lower dosages and less frequent administration is often possible. Modifications, such as lipidation, can be used to stabilize antibodies and to enhance uptake and tissue penetration (e.g., into the brain). A method for lipidation of antibodies is described by Cruikshank et al. ((1997) J. Acquired Immune Deficiency Syndromes and Human Retrovirology 14:193).

The UC-related gene product antagonist nucleic acid molecules can be inserted into vectors and used as gene therapy vectors. Gene therapy vectors can be delivered to a subject by, for example, intravenous injection, local administration (U.S. Pat. No. 5,328,470), or by stereotactic injection (see, e.g., Chen et al. (1994) Proc. Natl. Acad. Sci. USA 91:3054- 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

Pharmacogenomics

Agents, or modulators that have a stimulatory or inhibitory effect on activity or expression of a UC-related gene product polypeptide as identified by a screening assay described herein, can be administered to individuals to treat (prophylactically or therapeutically) disorders associated with aberrant activity of the polypeptide. In conjunction with such treatment, the pharmacogenomics (i.e., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of a UC-related gene product polypeptide, expression of a UC-related gene product nucleic acid, or mutation content of a UC-related gene product gene in an individual can be determined to thereby select an appropriate agent(s) for therapeutic or prophylactic treatment of the individual.

Pharmacogenomics deals with clinically significant hereditary variations in the response to drugs due to altered drug disposition and abnormal action in affected persons. See, e.g., Linder (1997) Clin. Chem. 43(2): 254-266. In general, two types of pharmacogenetic conditions can be differentiated. Genetic conditions transmitted as a single factor altering the way drugs act on the body are referred to as "altered drug action." Genetic conditions transmitted as single factors altering the way the body acts on drugs are referred to as "altered drug metabolism." These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, a PM will show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Thus, the activity of a UC-related gene product polypeptide, expression of a nucleic acid encoding the polypeptide, or mutation content of a gene encoding the polypeptide in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a modulator of activity or expression of the polypeptide, such as a modulator identified by one of the exemplary screening assays described herein.

## Methods of Treatment

Also described are prophylactic and therapeutic methods of treating a subject at risk of (or susceptible to) a disorder or having a disorder associated with aberrant expression or activity of a UC-related gene product polypeptide and/or in which the UC-related gene product polypeptide is involved.

Also described is a method for modulating or treating at least one UC-related gene product related disease or condition, in a cell, tissue, organ, animal, or patient, as known in the art or as described herein, using at least one UC-related gene product antagonist.

Compositions of UC-related gene product antagonist may find therapeutic use in the treatment of UC or related conditions, such as Crohn's disease or other gastrointestinal disorders.

Also described is a method for modulating or treating at least one gastrointestinal, immune related disease, in a cell, tissue, organ, animal, or patient including, but not limited to, at least one of gastric ulcer, inflammatory bowel disease, ulcerative colitis, Crohn's pathology, and the like. See, e.g., the Merck Manual, 12th-17th Editions, Merck & Company, Rahway, NJ (1972, 1977, 1982, 1987, 1992, 1999), Pharmacotherapy Handbook, Wells et al., eds., Second Edition, Appleton and Lange, Stamford, Conn. (1998, 2000).

Disorders characterized by aberrant expression or activity of the UC-related gene product polypeptides are further described elsewhere in this disclosure.

### 1. Prophylactic Methods

Also described is a method for at least substantially preventing in a subject, a disease or condition associated with an aberrant expression or activity of a UC-related gene product polypeptide, by administering to the subject an agent that modulates expression or at least one activity of the polypeptide. Subjects at risk for a disease that is caused or contributed to by aberrant expression or activity of a UC-related gene product can be identified by, for example, any or a combination of diagnostic or prognostic assays as described herein. Administration of a prophylactic agent can occur prior to the manifestation of symptoms characteristic of the aberrancy, such that a disease or disorder is prevented or, alternatively, delayed in its progression. Depending on the type of aberrancy, for example, an agonist or antagonist agent can be used for treating the subject. The appropriate agent can be determined based on screening assays described herein.

### 2. Therapeutic Methods

Also described are methods of modulating expression or activity of UC-related gene or gene product for therapeutic purposes. The modulatory method involves contacting a cell with an agent that modulates one or more of the activities of the polypeptide. An agent that modulates activity can be an agent as described herein, such as a nucleic acid or a protein, a naturally-occurring cognate ligand of the polypeptide, a peptide, a peptidomimetic, or other small molecule. In one embodiment, the agent stimulates one or more of the biological activities of the polypeptide. In another embodiment, the agent inhibits one or more of the biological activities of the UC-related gene or gene product polypeptide. Examples of such inhibitory agents include antisense nucleic acid molecules and antibodies and other methods described herein. These modulatory methods can be performed in vitro (e.g., by culturing the cell with the agent) or, alternatively, in vivo (e.g., by administering the agent to a subject). As such, the present invention provides methods of treating an individual afflicted with a disease or disorder characterized by aberrant expression or activity of a UC-related gene product polypeptide. In one embodiment, the method involves administering an agent (e.g., an agent identified by a screening assay described herein), or combination of agents that modulate (e.g., up-regulates or down-regulates) expression or activity. Inhibition of activity is desirable in situations in which activity or expression is abnormally high or up-regulated and/or in which decreased activity is likely to have a beneficial effect.

While having described the invention in general terms, the embodiments of the invention will be further disclosed in the following examples which should not be construed as limiting the scope of the claims.

**EXAMPLE 1: Sample Analysis by Using Nucleic Acid Microarrays Colon biopsies from infliximab treated ulcerative colitis patients Sample collection and RNA isolation**

Patients with moderate to severe active UC were randomly assigned 1:1:1 to intravenous placebo or infliximab (anti-TNF antibody) at a dose of 5 or 10 mg/kg at 0, 2, 6 and every 8 weeks thereafter. Colonoscopic punch biopsies were obtained from disease tissues at weeks 0 (prior to therapy), 8, and 30 and kept frozen until RNA preparation. RNA isolated from the biopsy samples was subsequently used for Affymetrix (oligonucleotide) microarray analysis. One hundred and twenty-three colon biopsy samples were collected from 49 subjects in this study. Gene expression profiles from 17 samples collected at week 0 (prior to treatment) from 16 subjects (there were 2 samples collected from one of the subjects - as a result of duplicative biopsy procedures) who responded to infliximab treatment in both 5 and 10mg/kg dose groups at both weeks 8 and 30 were compared to that of 6 samples from week 0 of non-responders across both dose groups at both time points as described herein. Treatment responders showed a marked clinical improvement following therapy defined by a decrease from baseline Mayo score by at least 3 points and at least 30% with an accompanying decrease in rectal bleeding sub-score of at least 1 point or an absolute rectal bleeding sub-score of 0 or 1. Clinical response was assessed at weeks 8 and 30 post-treatment.

Total RNA was isolated with an RNeasy mini kit according to the manufacturer's instructions (Qiagen Inc., Valencia, CA). The colon biopsy samples were lysed and homogenized in the presence of 600 μL of GITC (guanidine isothiocyanate)-containing buffer, which immediately inactivates RNase to ensure isolation of intact RNA. 600 μL of 70% ethanol was added to provide appropriate binding conditions and the sample was then applied to an RNeasy mini spin column where the total RNA binds to the membrane and contaminants were efficiently washed away. High-quality RNA was then eluted in 30 μL of water. RNA quality and quantity was analyzed with 2100 Bioanalyzer (Agilent Technologies Inc., Palo Alto, CA).

**Microarray Data Analysis**

Microarray analysis was performed on GeneChip Human Genome U133 Plus 2.0 arrays that allow the analysis of the expression level of more than 47,000 transcripts and variants, including 38,500 well-characterized human genes. RNA amplification, target synthesis and labeling, chip hybridization, washing and staining were performed in accordance with the manufacturer's protocol (Affymetrix, Santa Clara, CA). The GeneChips were scanned using the GeneChip Scanner 3000. The data were analyzed with GCOS 1.4 (GeneChip Operating System) using Affymetrix default analysis settings and global scaling as normalization method. The trimmed mean target intensity of each array was arbitrarily set to 500.

Data quality was assessed by hybridization intensity distribution and Pearson's correlation in Partek Pro software version 6.1 (Partek Inc., St. Charles, MO), and was deemed good except 2 samples, E36507_P43_5mg/kg_W30 & E36498_P39_placebo_W8. These samples were regarded as outliers and removed from data analysis.

Using GeneSpring™ software version 7.2 (Agilent Technologies, Palo Alto, CA), the intensity for probe set was normalized across all samples. Each measurement was divided by the median of all measurements in that sample. The intensity of a probe set was then normalized to the median intensity of that probe set in the control group. The control groups in this study were all 45 week 0 samples. Normalized intensity of probe set A in sample X was calculated as following:

$$\frac{\text{(Signal intensity of probe set A in sample X)}}{\text{(Median intensity of all measurements in sample X) x (Median intensity of probe set A across all week-0 samples)}}$$

Using Partek Pro 6.2, statistical analysis identified significant differences between responders and nonresponders using log-2 transformed normalized intensities. Samples from subjects of the same clinical response in both 5 and 10mg/kg dose groups at both weeks 8 and 30 were combined to increase the statistical power. ANOVA was conducted between responders and non-responders, using samples collected at week 0 (prior to treatment). Statistically significant differences were determined after applying a false discovery rate (FDR) of 10% for multiple testing correction.

Classification of infliximab responsiveness for each patient sample was generated with the 'K-Nearest Neighbors' algorithm, using GeneSpring™ 7.2. A classifier containing transcripts showing significant differential expression between responders and non-responders prior to IFX treatment was evaluated by leave-one-out cross-validation for its efficiency of classification. A p-value was calculated to measure the probability that a test sample was predicted as belonging to one class by chance. And a p-value ratio was defined as the p-value of the first best class relative to that of the next best class.

**Microarray Results**

Biopsies taken from infliximab treatment responders and non-responders at week 0 allowed an understanding of the potential mechanism underlying treatment response and non-response in UC, and thus predicting clinical response prior to treatment. The baseline responder samples analyzed were taken from patients who showed a marked clinical improvement following infliximab therapy based on assessment done at weeks 8 and 30 post-treatment, as defined by a decrease from baseline in the total Mayo score by at least 3 points and at least 30% with an accompanying decrease in rectal bleeding sub-score of at least 1 point or an absolute rectal bleeding sub-score of 0 or 1. The non-responder samples were taken from patients who didn't achieve the treatment response as defined above.

Gene expression results from responders in both 5- and 10-mg/kg dose groups were compared to that of non-responders at week 0. A set of 19 genes that demonstrated significant changes between an infliximab responder vs. non-responder is listed in Tables 1 A and 1 B. Each differentially expressed gene is presented by the ratio of normalized hybridization intensity of infliximab treatment responder samples to that of non-responder samples. Each gene is defined by Gene Ontology (GO) terms for its associated biological process, cellular component, and molecular function.

Table 1A and Table 1 B. A set of 19 genes differentially expressed between infliximab responders and non-responders at the baseline.

Table 1A:

| SEQ ID NO: | GenBank Accession # | Gene Name | Ratio* | NR&-Avg.Raw | NR - StdErr | R^ - Avg.Raw | R^ - StdErr |
|---|---|---|---|---|---|---|---|
| 1 | BC018088 | LOC645158 | 7.81 | 3.54 | 0.92 | 24.47 | 2.79 |
| 2 | AK098724 | | 5.4 | 8.39 | 5.08 | 40.28 | 5.45 |
| 3 | D14134 | RAD51 | 2.47 | 43.45 | 10.98 | 96.85 | 6.91 |
| 4 | BE504242 | LOC158402 | 2.26 | 178.98 | 20.80 | 354.16 | 24.11 |
| 5 | NM_024704 | C20orf23 | 1.75 | 388.31 | 29.58 | 600.39 | 25.45 |
| 6 | AB037781 | FLJ10074, SCYL2 | 1.68 | 1467.75 | 189.43 | 2184.08 | 69.49 |
| 7 | AB046777 | ARID2 | 1.6 | 313.24 | 12.65 | 445.25 | 17.64 |
| 8 | AK026684 | CCDC126 | 1.58 | 569.56 | 50.83 | 798.16 | 24.61 |
| 9 | AW250952 | DPP3 | 1.57 | 858.76 | 64.18 | 1189.86 | 33.48 |
| 10 | NM_000097 | CPOX | 1.56 | 664.66 | 53.96 | 927.17 | 32.51 |
| 11 | NM_014789 | ZNF623 | 1.5 | 250.96 | 21.51 | 336.63 | 14.27 |
| 12 | BF130937 | FLJ38973 | 1.5 | 758.28 | 51.36 | 1013.49 | 33.46 |
| 13 | AB040948 | USP28 | 1.48 | 244.00 | 18.07 | 319.50 | 12.82 |
| 14 | NM_014517 | UBP1 | 1.41 | 2195.07 | 102.07 | 2740.86 | 83.86 |
| 15 | AL512766 | LOC56181 | 1.4 | 522.34 | 50.82 | 647.39 | 23.30 |
| 16 | NM_018195 | C11orf57 | 1.39 | 503.94 | 29.48 | 621.88 | 17.54 |
| 17 | U81802 | PIK4CB | 1.34 | 679.31 | 31.80 | 804.75 | 24.78 |
| 18 | L080168 | TG4B | .28 | 132.89 | 2.09 | 287.67 | 5.43 |

(continued)

| SEQ ID NO: | GenBank Accession # | Gene Name | Ratio* | NR&-Avg.Raw | NR - StdErr | R^ - Avg.Raw | R^ - StdErr |
|---|---|---|---|---|---|---|---|
| 19 | M_018959 | AZAP1 | .25 | 796.76 | 9.36 | 002.93 | 8.39 |
| R* stands for the ratio of the average normalized intensity of a transcript in responder samples vs. that of non-responder samples at baseline. | | | | | | | |

Table 1B

| SEQ ID NO: | Description | GO-Biological process | GO-Cellular component | GO-Molecular function |
|---|---|---|---|---|
| 1 | hypothetical protein LOC645158 | | | |
| 2 | CDNA FLJ25858 fis, clone TST09644 | | | |
| 3 | RAD51 homolog (RecA homolog, E. coli) (S. cerevisiae) | DNA repair; meiotic recombination; mitotic recombination | nucleus | ATP binding; DNA-dependent ATPase activity; damaged DNA binding; protein binding; protein homooligomerization activity |
| 4 | hypothetical protein LOC158403 | | | |
| 5 | chromosome 20 open reading frame 23, also known as kinesin-like motor protein C20orf23 sorting nexin 23 | intracellular signaling cascade | microtubule associated complex | ATP binding; motor activity |
| 6 | hypothetical protein FLJ10074, also known as SCY1-like 2 protein coated vesicle-associated kinase of 104 kDa | protein amino acid phosphorylation | | ATP binding; protein kinase activity; transferase activity |
| 7 | AT rich interactive domain 2 (ARID, RFX-like) | Regulation of transcription, DNA-dependent | nucleus | DNA binding; zinc ion binding |
| 8 | coiled-coil domain containing 126 | | extracellular region | transferase activity |
| 9 | dipeptidylpeptidase 3 | proteolysis | cytoplasm | aminopeptidase activity;metallopeptidase activity; zinc ion binding |
| 10 | coproporphyrinogen oxidase | heme biosynthesis | mitochondrion | coproporphyrinogen oxidase activity; oxidoreductase activity |
| 11 | zinc finger protein 623 | Regulation of transcription, DNA-dependent | nucleus | DNA binding; zinc ion binding |
| 12 | hypothetical protein FLJ38973 | | | |

(continued)

| SEQ ID NO: | Description | GO-Biological process | GO-Cellular component | GO-Molecular function |
|---|---|---|---|---|
| 13 | ubiquitin specific protease 28 | ubiquitin-dependent protein catabolism | | cysteine-type endopeptidase activity; hydrolase activity; ubiquitin thiolesterase activity |
| 14 | upstream binding protein 1 (LBP-1a) | regulation of transcription from Pol II promoter; viral genome replication | | transcription corepressor activity; transcription factor activity |
| 15 | Family with sequence similarity 54, member B | | Extracellular region endoplasmic reticulum endoplasmic reticulum Membrane Membrane | Calcium ion binding selenium binding |
| 16 | Chromosome 11 open reading frame 57 | | | |
| 17 | phosphatidylinositol 4-kinase, catalytic, beta polypeptide | phosphatidylinositol biosynthesis; receptor mediated endocytosis;signal transduction | Cytoplasm, endoplasmic reticulum, endosome | phosphotransferase activity, alcohol group as acceptor |
| 18 | ATG4 autophagy related 4 homolog B (S. cerevisiae) | Autophagic vacuole formation proteolysis ubiquitin cycle protein targeting to membrane transport autophagy Autophagy protein transport | cytoplasm, microtubule associated complex | Protein binding Protein binding Peptidase activity cysteine-type peptidase activity Cysteine-type peptidase activity Hysrolase activity |
| 19 | DAZ associated protein 1 | spermatogenesis | nucleus | RNA binding |

Since these genes passed the ANOVA test with a false discovery rate of 10% or less when the infliximab treatment responder samples were compared to the corresponding non-responder samples, the entire expression profile as detailed in Tables 1A and 1 B are therefore defined as the infliximab treatment response signature in UC prior to treatment. In this baseline response gene signature, all the genes were expressed at higher levels in infliximab treatment responder samples as compared with that in the non-responder samples.

These results are novel findings in that clinical response outcome to infliximab treatment in moderate to severe UC can be predicted prior to treatment via assessing gene expression levels of a panel of selective genes. The expression changes in a panel of genes as represented in Tables 1A and 1 B can constitute a classifier that serves as a biomarker profile indicative of the response of a subject to treatment at baseline prior to any treatment.

**Real Time PCR (TaqMan®) confirmation:**

In order to confirm the microarray finding by an independent means, Real Time PCR (TaqMan®) technology was employed. Two micrograms of total RNA in the volume of 100 µL was converted to cDNA in the presence of MultiScribe Reverse Transcriptase. The reaction was carried out by incubating for 10 minutes at 25°C followed by 30 minutes at 48°C. Reverse Transcriptase was inactivated at 95°C for 5 minutes. Twenty-five nanograms of cDNA per reaction were used in real time PCR with ABI 7900 system (Foster City, California). In the presence of AmpliTaq Gold DNA polymerase (ABI biosystem, Foster City, California), the reaction was incubated for 2 minutes at 50°C followed by 10 minutes at 95°C. Then, the reaction was run for 40 cycles at 15 seconds, at 95°C and 1 minute, 60°C per cycle. The housekeeping gene GAPDH (glyceraldehyde-3-phosphate dehydrogenase) was used to normalize gene expression. The TaqMan® results on a selected number of genes are consistent with the observation from the microarray analysis.

The TaqMan® results on a selected number of genes are summarized in Table 2. Overall, it is consistent with the observation from the microarray analysis. The data is reported as the relative fold change in expression (responder relative to non-responder). Microarray data is shown for comparison purposes.

Table 2. TaqMan validation data comparing infliximab responder samples to non-responder samples at week 0

| SEQ ID NO: | Gene Name | Microarray Ratio (R/NR)* | TaqMan[&] FC | L/FC | U/FC |
|---|---|---|---|---|---|
| 5 | C20orf23 | 2.27 | 1.77 | 1.16 | 2.71 |
| 8 | LOC90693 | 1.85 | 1.18 | -1.32 | 1.84 |
| 10 | CPOX | 1.80 | 1.21 | -1.71 | 2.49 |
| 13 | USP28 | 1.73 | 1.58 | 1.02 | 2.44 |
| 14 | UBP1 | 1.53 | 1.08 | -1.24 | 1.45 |

* Mean ratio of five responders (R) vs. four non-responders (NR) at week 0 by microarray
[&]Mean fold change of five responders vs. four non-responders at week 0 by Taqman, FC=mean fold change, L=lower fold change value, U=upper fold change value

**Cross-validation of the treatment response classifier**

Among the 19 genes (Tables 1A and 1 B) that differentiate the infliximab responders and non-responders at the baseline, the differential expression of five of them, C20orf23 (SEQ ID NO:5), LOC90693 (SEQ ID NO:8), CPOX (SEQ ID NO:10), USP28 (SEQ ID NO:13), and UBP1 (SEQ ID NO: 14) were tested and confirmed by TaqMan (Table 2). The efficiency of the two panels of genes for patient classification was further evaluated by leave-one-out cross-validation. Classification was generated by the 'K-Nearest Neighbors' algorithm to cross-validate 22 baseline subjects (16 responders and six non-responders based on the assessment at both weeks 8 and 30 post-treatment) for the infliximab responsiveness. The panel of 19 or 5 genes achieved the same classification efficiency, by correctly classifying 21 patients (16 responders and five non-responders) and misclassifying one non-responder, with 100% of sensitivity and 83.3% specificity for prediction of treatment responsiveness at baseline (Table 3).

Table 3. Classification of the 16 responders and 6 non-responders for the infliximab responsiveness at baseline in UC

| **Subject** | **True Value** | **Prediction** | **P value ratio*** | **R - P value** | **NR - P value** |
|---|---|---|---|---|---|
| Subject 1 | NR | NR | 0.0114 | 1 | 0.0114 |
| Subject 29 | NR | NR | 0.000295 | 1 | 0.000295 |
| Subject 11 | NR | R | 0.645 | 0.55 | 0.852 |
| Subject 28 | NR | NR | 0.000295 | 1 | 0.000295 |
| Subject 32 | NR | NR | 0.000295 | 1 | 0.000295 |
| Subject 34 | NR | NR | 0.000295 | 1 | 0.000295 |
| Subject 13 | R | R | 0.0922 | 0.0922 | 1 |
| Subject 16 | R | R | 0.0922 | 0.0922 | 1 |
| Subject 38 | R | R | 0.0922 | 0.0922 | 1 |
| Subject 40 | R | R | 0.0922 | 0.0922 | 1 |
| Subject 9 | R | R | 0.0922 | 0.0922 | 1 |
| Subject 15 | R | R | 0.0922 | 0.0922 | 1 |
| Subject 17 | R | R | 0.0922 | 0.0922 | 1 |
| Subject 2 | R | R | 0.0922 | 0.0922 | 1 |
| Subject 24 | R | R | 0.0922 | 0.0922 | 1 |

(continued)

| Subject | True Value | Prediction | P value ratio* | R - P value | NR - P value |
|---|---|---|---|---|---|
| Subject 27 | R | R | 0.0922 | 0.0922 | 1 |
| Subject 36 | R | R | 0.0922 | 0.0922 | 1 |
| Subject 12 | R | R | 0.0922 | 0.0922 | 1 |
| Subject 21 | R | R | 0.467 | 0.424 | 0.908 |
| Subject 22 | R | R | 0.0922 | 0.0922 | 1 |
| Subject 43 | R | R | 0.0922 | 0.0922 | 1 |
| Subject 5 | R | R | 0.0922 | 0.0922 | 1 |
| *P value ratio is defined as the p-value (probability that the test sample is predicted as belonging to one class by chance) of the first best class relative to that of the next best class. | | | | | |

**Utility of the response signature.** The response signature for infliximab treatment in UC described herein can be assessed and used as described below.

1) Colonoscopic biopsy samples are obtained from lesional sites of patients with active UC (or Crohn's or related diseases and disorders). RNA will then be isolated from the biopsy samples and subjected to real time RT-PCR analysis. One microgram of total RNA in the volume of 50 μl is converted to cDNA in the presence of MultiScribe Reverse Transcriptase (ABI biosystem, Foster City, California). The reaction is carried out by incubating for 10 minutes at 25°C followed by 30 minutes at 48°C. Reverse Transcriptase is inactivated at 95°C for 5 minutes. Twenty-five nanograms of cDNA per reaction are used in real time PCR with ABI 7900 system (Foster City, California). In the presence of AmpliTaq Gold DNA polymerase (ABI biosystem, Foster City, California), the reaction is incubated for 2 minutes at 50°C followed by 10 minutes at 95°C. Then the reaction is run for 40 cycles at 15 seconds, at 95°C and 1 minute, 60°C per cycle using primer/probe sets specific for the genes in the response signature. House keeping genes, such as GAPDH or actin, will be used as internal calibrators. The relative change in gene expression is calculated using the delta-delta Ct method described by Applied Biosystems using values in the non-responder samples as the calibrator or comparator.
2) If a similar gene expression profile meets the parameters of the gene profile signature for a type of therapy, i.e., one or more of the 5 or 19 signature genes in the profiles described above show expression levels predictive of responders in relation to non-responders, the patient is considered a likely treatment responder to the therapy. In which case, the patient will be treated with the therapy.
3) If the gene expression profile does not meet the parameters of the gene profile signature for responder, i.e., lower expression level, then the patient is defined as a likely treatment non-responder. In which case, the patient may not be treated with the therapy. This enables a patient to avoid a type of therapy earlier after being deemed a non-responder. This can allow the patient to receive a different type of therapy.

Comparison method in relation to reference standard:

Total RNA is to be analyzed on a gene chip array for the expression intensities of the 19-gene panel listed in Tables 1A and 1B or the 5-gene panel listed in Table 2. The following procedures are exemplary of a method of evaluating members of a gene panel of the invention against a reference standard in order to compare values of the 19- or 5-gene panel members:

1. Total RNA is extracted from a biopsy sample from a prospective UC (or related disorder) patient before treatment and the total RNA quantity and quality is assessed as specified above in Example 1.
2. Total RNA is run in duplicate on three separate identical gene chip arrays, e.g., GeneChip Human Genome U133 Plus 2.0 arrays as follows:

a. RNA amplification, target synthesis and labeling, chip hybridization, washing and staining are performed according to the manufacturer's protocol, e.g., Affymetrix, Santa Clara, CA.
b. The GeneChips are scanned using, e.g., the GeneChip Scanner 3000.
c. The data is analyzed with, e.g., GCOS 1.4 (GeneChip Operating System) using Affymetrix default analysis settings and global scaling as normalization method, with the trimmed mean target intensity of each array arbitrarily set to 500.

d. The data quality is determined by correlating the data of each gene among the duplicates and across the three arrays.

i. A correlation coefficient > 0.9 should be achieved.

e. An average intensity value is calculated with a standard error representing the variability.

f. The patient should respond to treatment with an anti-TNF$\alpha$ antibody (e.g., infliximab) if:

i. The average intensity value is equal to or above X for each gene probe set (Table 4); or

ii. The average intensity value for the five (or 19) gene panel is equal to or above X (Table 4).

g. The patient should not respond to anti-TNF$\alpha$ antibody (e.g., infliximab) treatment if:

i. The average intensity value is below Y for each gene probe set (Table 4); or

ii. The average intensity value for the five (or 19) gene panel is below Y (Table 4).

Although illustrated and described above with reference to certain specific embodiments, the present invention is nevertheless not intended to be limited to the details shown. Rather, the present invention is directed to the UC-related genes and gene products. Polynucleotides, antibodies, apparatus, and kits disclosed herein and uses thereof, and methods for predicting responsiveness to treatment and controlling the levels of the UC-related biomarker genes, and various modifications may be made in the details within the scope and range of equivalents of the claims.

## Claims

**1.** A method for predicting the suitability of treatment with a target therapy for a gastrointestinal-related disorder in a subject, comprising:

a) preparing a sample of nucleic acids from a specimen obtained from the subject;
b) contacting the sample with a panel of nucleic acid segments consisting of at least a portion of 2 members from the group consisting of the nucleotide sequences corresponding to SEQ ID NOS:1-19 to detect levels of the panel segments, wherein the panel includes the nucleotide sequence corresponding to SEQ ID NO:1;
c) evaluating the sample against a reference standard to determine the magnitude of change in the amounts of at least 2 members present in the sample; and
d) correlating the magnitude of change with the suitability of treatment with the target therapy for the gastrointestinal-related disorder,

wherein the subject is a patient having ulcerative colitis and the target therapy is an anti-TNF$\alpha$ antibody, for example infliximab; and
the sample is from a subject providing the sample prior to administration of the therapy.

**2.** The method of claim 1, wherein the reference standard is from a colon biopsy from an untreated ulcerative colitis patient, a responder to the target therapy, or a non-responder to the target therapy.

**3.** The method of claim 1, wherein the collection is an array of nucleic acid segments.

**4.** The method of claim 1, wherein the evaluating step comprises:

i) evaluating the sample against a reference standard to determine the magnitude of change in the amounts of at least 5 members from the group consisting of the nucleotide sequences corresponding to SEQ ID NOS:1-19; or
ii) evaluating the sample against a reference standard to determine the magnitude of change in the amounts of at least 10 members from the group consisting of the nucleotide sequences corresponding to SEQ ID NOS:1-19; or
iii) evaluating the sample against a reference standard to determine the magnitude of change in the amounts of at least 15 members from the group consisting of the nucleotide sequences corresponding to SEQ ID NOS:1-19; or

iv) evaluating the sample against a reference standard to determine the magnitude of change in the amounts of all members from the group consisting of the nucleotide sequences corresponding to SEQ ID NOS:1-19.

**5.** The method of claim 1, wherein the sample comprises a colon biopsy sample.

**6.** The method of claim 1, wherein at least one member from the panel is selected from the group consisting of genes for proteins involved in nucleotide acid binding, ATP binding, transferase activity, proteolysis, oxidoreductase activity, ubiquitin thiolesterase activity, replication of proteins, signal transduction, and regulation of transcription.

**7.** An array-based testing method for predicting the suitability of treatment with a target therapy for ulcerative colitis in a patient, comprising:

a) preparing a mixture of nucleic acids from a specimen obtained from the patient;

b) labeling said specimen nucleic acids with a detectable marker to form a sample;

c) contacting the sample with an array comprising a plurality of nucleic acid segments, wherein each nucleic acid segment is immobilized to a discrete and known address on a substrate surface of the array, wherein at least two members of a ulcerative colitis-related gene panel consisting of the nucleotide sequences corresponding to SEQ ID NOS: 1-19 are identified as features of the array by address, and wherein said array further comprises at least one calibration nucleic acid at a known address on the substrate, and wherein the panel includes the nucleotide sequence corresponding to SEQ ID NO:1;

d) determining the degree of binding of the specimen nucleic acids to the nucleic acid segments; and

e) comparing the degree of binding to a reference standard to enable an assessment of the suitability of treatment,

wherein the therapy comprises an antagonist of TNF$\alpha$, for example an antibody to TNF$\alpha$, such as infliximab; and the specimen is from a patient providing the sample prior to administration of the therapy.

**8.** The method of claim 7, wherein the performing step comprises evaluating the sample against a reference standard to determine the magnitude of change in the amounts of at least two of the members of the nucleotide sequences corresponding to SEQ ID NOS:1-19.

**9.** The method of claim 7, wherein the specimen is from a colon biopsy of a patient selected from the group consisting of patients suspected of having ulcerative colitis and patients diagnosed with ulcerative colitis not undergoing treatment.

**10.** The method of claim 7, wherein the members of the gene panel are selected from the group consisting of genes for proteins involved in nucleotide acid binding, ATP binding, transferase activity, proteolysis, oxidoreductase activity, ubiquitin thiolesterase activity, replication of proteins, signal transduction, and regulation of transcription.

**11.** The method of claim 7, wherein the specimen comprises a colon biopsy sample or peripheral blood cells.

**12.** The method of claim 7, wherein the comparing the degree of binding step further comprises a stringent test of the similarity of feature intensity changes of the array of the ulcerative colitis-related gene panel.

**Patentansprüche**

**1.** Verfahren zur Vorhersage der Eignung einer Behandlung mit einer Ziel-Therapie für eine mit dem Gastrointestinaltrakt in Zusammenhang stehende Störung in einem Individuum, umfassend:

a) Herstellen einer Probe von Nukleinsäuren von einer aus dem Individuum erhaltenen Probennahme;

b) Inkontaktbringen der Probe mit einem Panel von Nukleinssäuresegmenten, die aus mindestens einem Anteil von 2 Mitgliedern aus der Gruppe bestehen, die aus den SEQ ID NO: 1-19 entsprechenden Nukleotidsequenzen besteht, um die Spiegel der Panelsegmente nachzuweisen, wobei das Panel die SEQ ID NO: 1 entsprechende Nukleotidsequenz enthält;

c) Untersuchen der Probe gegenüber einem Referenzstandard, um die Größenordnung der Veränderung in den Mengen von mindestens 2 in der Probe vorhandenen Mitgliedern zu bestimmen; und

d) Korrelieren der Größenordnung der Veränderung mit der Eignung der Behandlung mit der Ziel-Therapie für die mit dem Gastrointestinaltrakt in Zusammenhang stehende Störung,

wobei es sich bei dem Individuum um einen Patienten mit Colitis ulcerosa handelt und bei der Ziel-Therapie um einen Anti-TNFα-Antikörper, zum Beispiel Infliximab, und die Probe von einem Individuum stammt, das die Probe vor der Verabreichung der Therapie bereitstellt.

**2.** Verfahren nach Anspruch 1, wobei der Referenzstandard von einer Kolonbiopsie aus einem unbehandelten Colitis ulcerosa-Patienten, einem auf die Ziel-Therapie ansprechenden Individuum oder einem auf die Ziel-Therapie nicht ansprechenden Individuum stammt.

**3.** Verfahren nach Anspruch 1, wobei die Sammlung ein Array von Nukleinsäuresegmenten ist.

**4.** Verfahren nach Anspruch 1, wobei der Untersuchungsschritt Folgendes umfasst

i) Untersuchen der Probe gegenüber einem Referenzstandard, um die Größenordnung der Veränderung in den Mengen von mindestens 5 Mitgliedern aus der Gruppe, bestehend aus den SEQ ID NO: 1-19 entsprechenden Nukleotidsequenzen, zu bestimmen; oder
ii) Untersuchen der Probe gegenüber einem Referenzstandard, um die Größenordnung der Veränderung in den Mengen von mindestens 10 Mitgliedern aus der Gruppe, bestehend aus den SEQ ID NO: 1-19 entsprechenden Nukleotidsequenzen, zu bestimmen; oder
iii) Untersuchen der Probe gegenüber einem Referenzstandard, um die Größenordnung der Veränderung in den Mengen von mindestens 15 Mitgliedern aus der Gruppe, bestehend aus den SEQ ID NO: 1-19 entsprechenden Nukleotidsequenzen, zu bestimmen; oder
iv) Untersuchen der Probe gegenüber einem Referenzstandard, um die Größenordnung der Veränderung in den Mengen aller Mitglieder aus der Gruppe, bestehend aus den SEQ ID NO: 1-19 entsprechenden Nukleotidsequenzen, zu bestimmen.

**5.** Verfahren nach Anspruch 1, wobei die Probe eine Kolonbiopsieprobe umfasst.

**6.** Verfahren nach Anspruch 1, wobei mindestens ein Mitglied aus dem Panel aus der Gruppe ausgewählt ist, bestehend aus Genen für Proteine, die an Nukleotidsäurebindung, ATP-Bindung, Transferase-Aktivität, Proteolyse, Oxidoreduktase-Aktivität, Ubiquitinthiolesterase-Aktivität, Replikation von Proteinen, Signaltransduktion und Regulation der Transkription beteiligt sind.

**7.** Array-basiertes Testverfahren zum Vorhersagen der Eignung einer Behandlung mit einer Ziel-Therapie für Colitis ulcerosa in einem Patienten, umfassend:

a) Herstellen eines Gemischs von Nukleinsäuren aus einer von dem Patienten erhaltenen Probenentnahme;
b) Markieren der Probenentnahme-Nukleinsäuren mit einem nachweisbaren Marker, um eine Probe herzustellen;
c) Inkontaktbringen der Probe mit einem Array, der einer Mehrzahl von Nukleinsäuresegmenten umfasst, wobei jedes Nukleinsäuresegment an einer getrennten und bekannten Adresse auf einer Substratoberfläche des Arrays immobilisiert ist, wobei mindestens zwei Mitglieder eines mit Colitis ulcerosa in Zusammenhang stehenden Genpanels, bestehend aus den SEQ ID NO: 1-19 entsprechenden Nukleotidsequenzen, als Merkmale des Arrays anhand der Adresse identifiziert werden und wobei der Array zudem mindestens eine Kalibrierungs-Nukleinsäure an einer bekannten Adresse auf dem Substrat umfasst und wobei das Panel die SEQ ID NO: 1 entsprechende Nukleotidsequenz enthält;
d) Bestimmen des Grads der Bindung der Probenentnahme-Nukleinsäuren an die Nukleinsäuresegmente und
e) Vergleichen des Grads der Bindung an einen Referenzstandard, um eine Beurteilung der Eignung der Behandlung zu ermöglichen,

wobei die Therapie einen Antagonisten von TNFα, zum Beispiel einen Antikörper gegen TNFα, wie Infliximab, umfasst und
die Probenentnahme von einem Patienten stammt, der die Probe vor der Verabreichung der Therapie bereitstellt.

**8.** Verfahren nach Anspruch 7, wobei der Durchführungsschritt das Untersuchen der Probe gegenüber einem Referenzstandard umfasst, um die Größenordnung der Veränderung in den Mengen von mindestens zwei der Mitglieder der den SEQ ID NO: 1-19 entsprechenden Nukleotidsequenzen zu bestimmen.

**9.** Verfahren nach Anspruch 7, wobei die Probenentnahme aus einer Kolonbiopsie von einem Patienten stammt, der aus der Gruppe ausgewählt ist, bestehend aus Patienten, von denen vermutet wird, dass sie Colitis ulcerosa haben, und Patienten, bei denen Colitis ulcerosa diagnostiziert wurde, die keiner Therapie unterliegen.

**10.** Verfahren nach Anspruch 7, wobei die Mitglieder des Genpanels aus der Gruppe ausgewählt sind, bestehend aus Genen für Proteine, die an Nukleotidsäurebindung, ATP-Bindung, Transferase-Aktivität, Proteolyse, Oxidoreduktase-Aktivität, Ubiquitinthiolesterase-Aktivität, Replikation von Proteinen, Signaltransduktion und Regulation der Transkription beteiligt sind.

**11.** Verfahren nach Anspruch 7, wobei die Probenentnahme eine Kolonbiopsieprobe oder periphere Blutzellen umfasst.

**12.** Verfahren nach Anspruch 7, wobei der Schritt des Vergleichens des Grads der Bindung weiterhin einen stringenten Test der Ähnlichkeit von Merkmalsintensitätsveränderungen des Arrays des mit Colitis ulcerosa in Zusammenhang stehenden Genpanels umfasst.

**Revendications**

**1.** Procédé pour prédire le caractère approprié d'un traitement par une thérapie cible, pour un trouble associé aux troubles gastro-intestinaux chez un sujet, comprenant :

a) la préparation d'un échantillon d'acides nucléiques à partir d'un spécimen obtenu d'un sujet ;
b) la mise en contact de l'échantillon avec un panel de segments d'acides nucléiques consistant en au moins une portion de 2 membres du groupe consistant en les séquences nucléotidiques correspondant à SEQ ID NO:1 à 19 pour détecter les niveaux des segments du panel, le panel comprenant la séquence nucléotidique correspondant à SEQ ID NO:1 ;
c) l'évaluation de l'échantillon par comparaison avec un étalon de référence pour déterminer l'importance du changement des quantités d'au moins 2 membres présents dans l'échantillon ; et
d) la corrélation de l'importance du changement avec le caractère approprié du traitement par la thérapie cible pour le trouble associé aux troubles gastro-intestinaux,

dans lequel le sujet est un patient présentant une recto-colite hémorragique, et la thérapie cible étant un anticorps anti-TNF$\alpha$, par exemple l'infliximab ; et
l'échantillon provenant d'un sujet fournissant l'échantillon avant administration de la thérapie.

**2.** Procédé selon la revendication 1, dans lequel l'étalon de référence provient d'une biopsie du côlon d'un patient présentant une recto-colite hémorragique non-traitée, d'un répondeur à la thérapie cible, ou d'un non-répondeur à la thérapie cible.

**3.** Procédé selon la revendication 1, dans lequel la collection est une biopuce de segments d'acides nucléiques.

**4.** Procédé selon la revendication 1, dans lequel l'étape d'évaluation comprend :

i) l'évaluation de l'échantillon par référence à un étalon de référence pour déterminer l'importance du changement des quantités d'au moins 5 membres du groupe consistant en les séquences nucléotidiques correspondant à SEQ ID NO:1 à 19 ; ou
ii) l'évaluation de l'échantillon par comparaison avec un étalon de référence pour déterminer l'importance du changement des quantités d'au moins 10 membres du groupe consistant en les séquences nucléotidiques correspondant à SEQ ID NO:1 à 19 ; ou
iii) l'évaluation de l'échantillon par comparaison avec un étalon de référence pour déterminer l'importance du changement des quantités d'au moins 15 membres du groupe consistant en les séquences nucléotidiques correspondant à SEQ ID NO:1 à 19 ; ou
iv) l'évaluation de l'échantillon par comparaison avec un étalon de référence pour déterminer l'importance du changement des quantités de tous les membres du groupe consistant en les séquences nucléotidiques correspondant à SEQ ID NO:1 à 19.

5. Procédé selon la revendication 1, dans lequel l'échantillon comprend un échantillon de biopsie du côlon.

6. Procédé selon la revendication 1, dans lequel au moins un membre du panel est choisi dans le groupe consistant en les gènes pour les protéines impliquées dans la liaison des acides nucléotides, la liaison à l'ATP, l'activité de transférase, la protéolyse, l'activité d'oxydoréductase, l'activité d'ubiquitine thiolestérase, la réplication des protéines, la transduction du signal et la régulation de la transcription.

7. Méthode d'essai à base d'une biopuce, pour prédire le caractère approprié du traitement par une thérapie cible pour une recto-colite hémorragique chez un patient, comprenant :

a) la préparation d'un mélange d'acides nucléiques à partir d'un spécimen obtenu du patient ;
b) le marquage desdits acides nucléiques spécimen avec un marqueur détectable pour former un échantillon ;
c) la mise en contact de l'échantillon avec une biopuce comprenant une pluralité de segments d'acides nucléiques, chaque segment d'acide nucléique étant immobilisé sur une adresse discrète et connue sur la surface d'un substrat de la biopuce, au moins deux membres d'un panel de gènes associés à la recto-colite hémorragique, qui consistent en les séquences nucléotidiques correspondant à SEQ ID N0:1 à 19, étant identifiés comme étant des particularités de la biopuce par adresse, et ladite biopuce comprenant en outre un acide nucléique d'étalonnage à une adresse connue sur le substrat, et le panel comprenant la séquence nucléotidique correspondant à SEQ ID NO:1 ;
d) la détermination du degré de liaison des acides nucléiques du spécimen aux segments d'acides nucléiques ; et
e) la comparaison du degré de liaison à un étalon de référence, pour permettre une évaluation du caractère approprié du traitement,

dans laquelle la thérapie comprend un antagoniste du TNF$\alpha$, par exemple un anticorps contre le TNF$\alpha$, tel que l'infliximab ; et
le spécimen provenant d'un patient fournissant l'échantillon avant administration de la thérapie.

8. Procédé selon la revendication 7, dans lequel l'étape de mise en oeuvre comprend l'évaluation de l'échantillon par comparaison avec un étalon de référence pour déterminer l'importance du changement des quantités d'au moins deux des membres des séquences nucléotidiques correspondant à SEQ ID NO:1 à 19.

9. Procédé selon la revendication 7, dans lequel le spécimen provient d'une biopsie du côlon d'un patient choisi dans le groupe consistant en les patients suspectés de présenter une recto-colite hémorragique et les patients diagnostiqués avec une recto-colite hémorragique et ne subissant pas le traitement.

10. Procédé selon la revendication 7, dans lequel les membres du panel de gènes sont choisis dans le groupe consistant en les gènes pour les protéines impliquées dans la liaison des acides nucléotides, la liaison à l'ATP, l'activité de transférase, la protéolyse, l'activité d'oxydoréductase, l'activité d'ubiquitine thiolestérase, la réplication des protéines, la transduction du signal et la régulation de la transcription.

11. Procédé selon la revendication 7, dans lequel le spécimen comprend un échantillon de biopsie du côlon ou des cellules du sang périphérique.

12. Procédé selon la revendication 7, dans lequel l'étape de comparaison du degré de liaison comprend en outre un test stringent portant sur la similitude des changements d'intensité de particularités de la biopuce du panel de gènes associés à une recto-colite hémorragique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A
- US 4683202 A
- WO 0194630 A2
- US 20020006622 A
- WO 9318186 A
- US 6344316 B
- US 6197503 B
- US 6174684 B
- US 6159685 A
- US 6156501 A
- US 6093370 A
- US 6087112 A
- US 6087103 A
- US 6087102 A
- US 6083697 A
- US 6080585 A
- US 6054270 A
- US 6048695 A
- US 6045996 A
- US 6022963 A
- US 6013440 A
- US 5959098 A
- US 5856174 A
- US 5843655 A
- US 5837832 A
- US 5770456 A
- US 5723320 A
- US 5700637 A
- US 5695940 A
- US 5556752 A
- US 5143854 A
- WO 9951773 A
- WO 9909217 A
- WO 9746313 A
- WO 9617958 A
- WO 8910977 A
- US 5843767 A
- WO 04002417 A
- US 5328470 A
- WO 60914908 A


### Non-patent literature cited in the description


- Current Protocols in Immunology. John Wiley & Sons, Inc, 1994
- **COLLIGAN et al.** Current Protocols in Polypeptide Science. John Wiley & Sons, 1997
- Computational Molecular Biology. Oxford University Press, 1988
- Biocomputing:Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. M Stockton Press, 1991
- **CARILLO, H. ; LIPMAN, D.** *Siam J. Applied Math.,* 1988, vol. 48, 1073
- PCR PROTOCOLS, A GUIDE TO METHODS AND APPLICATIONS. Academic Press, 1990
- PCR STRATEGIES. Academic Press, Inc, 1995
- **WU.** *Genomics,* 1989, vol. 4, 560
- **LANDEGREN.** *Science,* 1988, vol. 241, 1077
- **BARRINGER.** *Gene,* 1990, vol. 89, 117
- **KWOH.** *Proc. Natl. Acad. Sci. USA,* 1989, vol. 86, 1173
- **GUATELLI.** *Proc. Natl. Acad. Sci. USA,* 1990, vol. 87, 1874
- **SMITH.** *J. Clin. Microbiol.,* 1997, vol. 35, 1477-1491
- **BURG.** *Mol. Cell. Probes,* 1996, vol. 10, 257-271
- **BERGER.** *Methods Enzymol.,* 1987, vol. 152, 307-316
- **SOOKNANAN.** *Biotechnology,* 1995, vol. 13, 563-564
- **CRAIG.** *Hum. Genet.,* 1997, vol. 100, 472-476
- **RAUCH.** *J. Biochem. Biophys. Methods,* 2000, vol. 44, 59-72
- **JOHNSTON.** *Curr. Biol.,* 1998, vol. 8, R171-174
- **SCHUMMER.** *Biotechniques,* 1997, vol. 23, 1087-1092
- **KERN.** *Biotechniques,* 1997, vol. 23, 120-124
- **SOLINAS- TOLDO.** *Genes, Chromosomes & Cancer,* 1997, vol. 20, 399-407
- **BOWTELL.** *Nature Genetics,* 1999, vol. 21, 25-32
- **EPSTEIN.** *Current Opinion in Biotech.,* 2000, vol. 11, 36-41

- **MENDOZA.** *Biotechniques,* 1999, vol. 27, 778-788
- **LUEKING.** *Anal. Biochem.,* 1999, vol. 270, 103-111
- **DAVIES.** *Biotechniques,* 1999, vol. 27, 1258-1261
- **CRUIKSHANK et al.** *J. Acquired Immune Deficiency Syndromes and Human Retrovirology,* 1997, vol. 14, 193
- **CHEN et al.** *Proc. Natl. Acad. Sci. USA,* 1994, vol. 91, 3054-3057
- **LINDER.** *Clin. Chem.,* 1997, vol. 43 (2), 254-266
- Merck Manual. Merck & Company, 1972
- Pharmacotherapy Handbook. Appleton and Lange, 1998

SEQUENCE LISTING

<110> CENTOCOR, INC.

<120> MARKERS AND METHODS FOR ASSESSING AND TREATING ULCERATIVE COLITIS AND RELATED DISORDERS USING A 19 GENE PANEL

<130> CEN5178PCT

<140> TO BE ASSIGNED
<141> 2008-04-28

<150> 60/914,908
<151> 2007-04-30

<160> 19

<170> Fast SEQ f or Windows Ver si on 4.0

<210> 1
<211> 506
<212> DNA
<213> Homo sapi ens

<220>
<223> (81) (136) (250) (273) (274) (280) (281) (283) (284) (285) (288) (289) (290) (298) (301) (307) (311) (312) (313) (318) (336) (347) (363) (366) (382) (390) (392) (393) (394) (395) (413) (439)

<400> 1

gcagt at act gccat at agt gt gt gt gt gt gt aaaacct a ggt ct t gat t t t ct t at t ag 60
t gt gaaat ac t ct at t t t t a naaaaat aga gat agagt ct cact at gt t g cccaggt t gg 120
t ct caaact c t gggcnt caa gt gat cct cc t gcct t ggct t cccaaagt gct gggact a 180
cagacgt gag ccaccct gcc t ggccagct a t t t t ct aat g aaat ct at t t t ct act gcaa 240
aat ct t gt t n t gaagt agaa t caaaagagt t t nggggt n ngnnngt nnn gggt at t ngt 300
nggggnt t t t nnncat cnat agcact t gt t act t t ngaac aagt agnaaa agct t t ct gt 360
agnt gnt t ca t t t accagaa angaat at t n gnnnncat gg t at at t at t t ccnct t t t ct 420
aaat gt t ggg aacat t t t nc cat aat cat t act caat cat aact t gt gt t t aacct at aa 480
t gcct aaggc t at t ct gaat t t t t gt 506

<210> 2
<211> 541
<212> DNA
<213> Homo sapi ens

<400> 2

agct gt cagt aaaaat gct g t gcaat at gt ggat t cat gt cacaaat t t a aacct ct gca 60
t t t at t cagc aagt t t ggaa t acct t t t t g t agaat at ac aaagggat gt t t cacacct a 120
t aaaagcct a t agt aaaaaa gt t ggat aca ct ct t gt t gt agaat ct aca aagagacat t 180
t t gaagt t ca t t gaggt ct a t agt gaaaaa ccaaat at cc agt gat agaa act at aaaga 240
agct at ct ct gaaaat act t t at gat gt gt gat t t cat t t cacaagt t ga act t t t ct t t 300
t gat t caaca gcaggt t gga aacact ct t t t gt aaaat ct acaaagaaac at t t t ggagt 360
ccat t gaggc ct at agt gaa aaact gaat a t t t cgcct t g aaaaccagaa acaagct at c 420
t gt gaaaat g ct t t gt gat g gaagat t t at ct cacagagt t aaacgt t t g t at t gat t ca 480
gct ggct ggc aacact ct t t t t gt aaaat c t acaat ggga cat t t cagag accat t gaag 540
t 541

<210> 3
<211> 371
<212> DNA
<213> Homo sapi ens

<400> 3

gaat gggt ct gcacagat t c t t t t t t t ct g t cagt aaaac t ct caagcag gt t t t t aagt 60
t gt ct gt ct g aat gat ct t g t gt aagggt t t ggt t at gga gt ct t gt gcc aaacct act a 120
ggccat t agc cct t caccat ct acct gct t ggt ct t t cat t gct aagact aact caagat 180

aat cct agag t ct t aaagca t t t caggcca gt gt ggt gt c t t gcgcct gt act cccagca 240
ct t t gggagg ccgaggcagg t ggat cgct t gagccaggag t t t t aagt cc agct t ggcca 300
agat ggt gaa at cccat ct c t acaaaaaat gcagaact t a at ct ggacac act gt t acac 360
gt gcct gt ag t 371

<210> 4
<211> 357
<212> DNA
<213> Homo sapi ens

<400> 4

cct cagcgcc act gagt gct gt t gt at t gt t aat ct t gat t aat ct ggt a t gt gaagaag 60
gat gt t t caa t gt t t t gt ct gt gt ct ct ct gat t at t t t g ct t gt t gat t ggccagt t gt 120
t aat t ct gt c t t t gt gagt t gt ct t t t t ct cagt act t gg cct at t t gt c t t t gat t t ga 180
aaaagct ct t t at gt t gt ag t cat t t t aat t cct gt cat a t gt t t t gt aa acaat t t t t c 240
gagt t cat aa t t t t t caat c t t gt t t gt at t at at t t t gc cacacaaaaa t t t t aaat t t 300
gt at agt caa at t t at cagt ct t t t t cct t at gt t ggacc t t ct aat ct c aaggt ac 357

<210> 5
<211> 472
<212> DNA
<213> Homo sapi ens

<400> 5

```
gggat t t t t t  gat ccaat ca  t gact gt agt  ct t ccat gct  t gacaaat t g  gat gt agaca  60
acat t act t a  aaact t ct at  aaat ccct ac  aat t aggat a  t t t at t t aac  ct t gaat at t  120
caagaacat t  ct cccaaat c  t aaat ggct a  ct gt gcat t c  t t gagct t t t  t ct gct aagc  180
acaaaat gaa  cgcaaagct a  aat gcat at t  t t t aagt at t  at t cacat t t  t t t gt t acag  240
aat ct at t gg  at ct t t ggct  ggaaaact ag  aat t t at agc  agt t t at t aa  t gat acct t a  300
aat t act cag  gact t aat gt  agcat t gcac  t t ct gt gt ac  agt aaaact g  ct t t gt t t t a  360
ct aaagagaa  aaat gt gagt  ggaaaaaat a  t gt at gt gt t  at at act caa  at gt at at aa  420
t t ct at ct at  agat t t at at  at gt at acat  t ct gt acagt  agt t ccat ca  aa  472
```

<210> 6
<211> 514
<212> DNA
<213> Homo sapi ens

<400> 6

```
t acacct t t t  t t ct act t ca  ggt at t t t at  t t caaccat t  t ccat caat t  gaact gt t ac  60
cat t gcct t t  t t ct gt t gag  aaat t gcct c  t gaaaaat ag  t gct at t t t t  cagct t aagt  120
gt t ct t aagt  gaat gaaat t  t t caaagt ac  t agat cacct  t aaaat t at t  t cacgt act g  180
aagacaat t a  agt ccgt t at  gt t t agagt a  gaaaat gt t t  aggt t aaaga  gcat ct gt ca  240
acagaat ct a  caaaaaagat  t ccct t gcat  t t gaat t agt  t ct ct at t ct  cct at t gct a  300
aat gt gt gat  at at agagag  gat gt at aaa  aggaaat gga  aat agact at  gt act t gt ct  360
ggt t t t t gt t  t gt t t t t at t  t t ggaat gct  t at aagcct c  ct t t acact g  aat aaggaag  420
t agt t t t t gt  t t t ct t t t ga  cct gt aaaat  acct cacat g  gt t gt t t t t a  cacat gaaag  480
aaaaaggt at  at gcgaacat  acct gat at c  aaga  514
```

<210> 7
<211> 496
<212> DNA
<213> Homo sapi ens

<400> 7

```
gcct t t t aag  ct t cagt ct c  agcagagaat  t t cct aaat g  cgt t t gacct  aat gaaact g  60
at cat ggct t  cccact t agg  t t t t t ct t ct  t at agct t t a  t agaact at a  t aat aat at g  120
gact t gct gt  gt aat ggaat  t aaagt gct t  t t gcacaat a  agt t ct gcaa  aaccct ct ca  180
t t cat gaaaa  ggt gct cct t  gct agacaga  aact t gct ga  t t t acagt at  t gt t at t t t t  240
gt ct aaagt t  ct gt aaat ac  at gct t t aat  gt t at ct t t g  agaaat ct at  gt aaat aat a  300
t agt ct acaa  cat agagact  gt at aat t ct  gt gt t at at a  t gt gcct agt  gct ct gt t gg  360
cact caat aa  at t t t aagt a  acaaaat t ga  t aat cat at a  gcgaaggcat  at t t t t ct t c  420
caagct caag  t caggat t gt  gact at at at  t aat gagact  cagt aat cca  acccacacct  480
gagaact cgt  ct cat t  496
```

<210> 8
<211> 396
<212> DNA
<213> Homo sapi ens

<220>
<223> (61) (174)

<400> 8

agcacaggga accctaatct tgggtaattc tagtataaaa caaattatac ttttatttaa 60
ntttcccttg tagcaaatct aattgccaca tggtgcccta tatttcatag tatttattct 120
ctatagtaac tgcttaagtg cagctagctt ctagatttag actatataga attnagatat 180
tgtattgttc gtcattataa tatgctacca catgtagcaa taattacaat attttattaa 240
aataaatatg tgaaatattg tttcatgaaa gacagatttc caaatctctc ttctcttctc 300
tgtactgtct acctttatgt gaagaaatta attatatgcc attgccaggt agaagtttgg 360
aataattgtt tagtctctcc tattagatgt ggacat 396

<210> 9
<211> 490
<212> DNA
<213> Homo sapi ens

<220>
<223> (96) ( 172) (255) (449)

<400> 9

ggtgctgctg cgtaaggaat ctcggaagct cattgttcag cccaacactc gccttgaagg 60
ctcagacgtg cagcttctgg aatacgaggc gtcagntgct ggcctcatcc gatccttctc 120
tgagcgtttc ccagaggatg gacccgagtt ggaggagatc ctcacacagc tngccacagc 180
cgatgcccga ttctggaagg gccccagtga ggccccatct ggccaagctt gaggaagatg 240
tgtggccttg ccccnaattc catcagacca aggctgcaag tggccctcca ttcgtcactt 300
tccagcctgc caattgcttc ccctctgtga tctcatttca tctgcactgc catacgtgga 360
gtgagcaaga cagggcttac catcctgtct accagatgag gaaatggcag ttctgagaag 420
tcactggtct agatcccgca ggtggcacnt gacagctagg gttcaaaacg ttctcaccaa 480
atccaatgct 490

<210> 10
<211> 548
<212> DNA
<213> Homo sapi ens

<400> 10

attcttaatc tctgcatgtg ttacaattca gttatttctg tagtttgtaa actctaaagt 60
gacattacta ttattttaga gatgtctaag ttgtaatttt gatttttgtg gaaccattgt 120
ttgttaatgt tgggattctc tgcacttttg aatgtgaaag cttatatccc tgaattctga 180
tacttaagag ttttctattt cagacatctc tgtgtggaag ttgagactaa gaataatcct 240
agggatgtca tgaatttagg caatgtttct catttggaaa atgaaatgag aaataatttc 300
cttctttaaa gcaagtatat atagtatgag aaacttggag gcatttcata cacacatttc 360
ttaggaaaat ggacacattg aaaatgtcct cttttttata ttagagattc tgcagctctt 420
tgctcttaag agcaaatcac aacaggattc ttaatgtatg atttctttgt tcatatttat 480
gaatgtatta ttttatttgc ttcgtaataa agtttataag gaagagcatc tcatacatat 540
cattatcg 548

<210> 11
<211> 519
<212> DNA
<213> Homo sapi ens

<400> 11

aat gcggaag agt t agccag gcgt ggt ggc aggcgcct gt agaat cccag ct gct cggga 60
ggct gaggca ggagaat t gc t t gagcct gg gaggt ggagg t t t cagt gag ct gggat cgt 120
ggcat t gcac t ct agcct gg gcaaccaaga gt gaaact gt ct caaaaaac aact t t t at c 180
aat gt ct gca aaaagaaagt ct t ct gggat t t at agat ca at t t agggag aaat gacat t 240
t t aacaat t c t gagt t t t cc aat t gt t gaa cat ggt gt ac t gccccat t t at t t agat ct 300
gt t aat t t ct ct cagt t t gc agct ct caca t t t t gt t aaa t t cat gt at t t aat at t t ct 360
gcat gct at t gcaagt ggt a aggt t t t caa aaagct gt t t t ct agt t at t gct agt at at 420
agaaat gcat t agact t gt a cat t gat ct t gt at caagca act t agat ca gt t aact t at 480
t ct agt agct t t t t t ct aga t t ct t t agca t t t t ct at g 519

<210> 12
<211> 470
<212> DNA
<213> Homo sapi ens

<400> 12

aat t t t t cat t at at cagt c t t t t t gaaag gat caact t g gat aaaat aa at at at aat g 60
ct ct at t t gt t agagct ct a t t aaaaagga aacagat t cc at agat ct aa gt caat gt t t 120
ct ccagaagc at gat t t t gt ct gccaaaag aaaat agct c t ct t t ggcca aaat gcaaaa 180
t t acat t gct at aagaaaag t t acaaggga aagt t t gaag acacaaat ga t t t aat t t t g 240
gct caaaaac t gaat t t gct t aacact gct acat aat t t g ggt gaagt t t cct t ct gccc 300
gt t t t t ct t g acct agat aa at acact t t g agaaat ccag at ct aat aaa t gt caaccaa 360
cat t gacat t gt aat t gggt gat t acaat a aaaggt gagc agt t t gt t gt t t at t aat aa 420
t t agct t t t g caggt aat ga aat agcaggg aagt aacat g ct gct t t agg 470

<210> 13
<211> 274
<212> DNA
<213> Homo sapi ens

<220>
<223> (213)

<400> 13

accagaaaac ct cat act ca gt cct cagt g aaagaaagt a aagt at t aag gact t t agac 60
agaagagcat t gt gt aact t gact gaagat cat ccat t aa t agt t at t ag gcat t t aggt 120
aaaat t t t ct aat acct aaa aat t gt caaa aacagt caat agggct act g ct ggcccaaa 180
gaccat t t ag gt ccacct cc t ct t t t t t gc t cnt t t t t t t t t t t ct gt ga cagt t t cact 240
gt gt cgccca ggct ggcgt t cagt ggt gca at ct 274

<210> 14
<211> 455
<212> DNA
<213> Homo sapi ens

<400> 14

t t t t gagt t t gaaat ct ct c ccagaagaca aact act t ca gt gagt aaaa gct t t gacat 60
t t t at gt t t t at t cat aaag ggggt t aat t at t t gct aca aagaagcacg at ct at t t t c 120
at cat cgat t t gaaaat at c t gt aact cct at agat cct a t aggcagaga gt t t t cct t t 180
ct gact t t t t ccct t t gct t t cgt gt gacc acat gt t t t c t gt accagt c act ggggaaa 240
gaagt gagt t t at ct cgt t t gt t t t aaaag t t t t gct t gt ct at t t agca t t cct t t t t g 300
ggt ct caaga t t t at ggaac aat aaat gt c at t t aat gct gt gt gct t at t t t gaat t cc 360
t cat caggt t t t agaagcgg ggt aaaaat a ct t agat gct t at cagact t gaaat t at ac 420
t gagt ggcat t gaacgt gag t t t gt cccag t gaaa 455

<210> 15

<211> 560
<212> DNA
<213> Homo sapiens

<400> 15

```
gaccctatag ctggtctcac aagacacttt gtgcccagct gtcactcact ctcagcagct 60
tccttgcagc agagcagggc tgaggggaag gggctatgaa tgtttgtata cgtgttcaca 120
gggcacggaa aatcttatgc tgctccgtca taaacctaca ccaatgccca gcaatcaccc 180
tcctcacttc cttgtctaga tgtagaggtc aggctgctga accagccaac acatgggcta 240
ctgctgggaa gcctgggctg ttttttttct taaacacatt ttatattact gaacaaccaa 300
atctaccctc cacggccctg aggccttatc agttccactg attaaaaact ttctcttcca 360
cggactttaa gcccggtagg aaagagagag gaggaggggg aaagagcaaa ccatctttct 420
tccaggccct tgactgctcc tttgggctgg gccaaggttt gtatgtacca caccatgcat 480
gactcagatg ccctcaggtc cctttctcta tggtatgtat actgcttgtg cttgggttga 540
agcactacct gacattaaag                                             560
```

<210> 16
<211> 554
<212> DNA
<213> Homo sapiens

<400> 16

```
gcaattattt tgttcctgga gcaagttaaa tctttgttgg aaggagcttt ggccatatat 60
tttttagcat gcattgtttc tgtgccctga aagtacctga aaggttttaa gcacagactc 120
aggaaaatgt gccagtagaa caggccatct ccaggaaatt ggctctattt gggtcctgac 180
cttcccttcc tcccaagtta gcaggcttgt tcttttgcaa ggaatacaca tcttgccttt 240
tttttttttt tttttttgcc atgttttcct tttcttggtc atgtataagc aataaagctg 300

ttttttgttc ttcatctttc ttaaccccaa attttcttct atgccttagg cttcgatggt 360
tcttccaacc cccttaatat ggcttagggt ggtttttcaa aacctacaat cccccatttg 420
cactactggc catggaacat ttatttctag tgttcctgcc aatcagagat ctctatatta 480
aattctaaaa tgggattaaa agaagagttg gagaattcac acttattgag taactgatgt 540
catacaacct ggaa                                                   554
```

<210> 17
<211> 543
<212> DNA
<213> Homo sapiens

<400> 17

```
atcgtggaga tcatgcagca aggttctcag cttccttgct tccatggctc cagcaccatt 60
cgaaacctca aagagaggtt ccacatgagc atgactgagg agcagctgca gctgctggtg 120
gagcagatgg tggatggcag tatgcggtct atcaccacca aactctatga cggcttccag 180
tacctcacca acggcatcat gtgacacgct cctcagccca ggagtggtgg ggggtccagg 240
gcaccctccc tagagggccc ttgtttgaga aaccccaaac caggaaaccc cacctaccca 300
accatccacc caagggaaat ggaaggcaag aaacacgaag gatcatgtgg taactgcgag 360
agcttgctga ggggtgggag agccagctgt ggggtccaga cttgttgggg cttccctgcc 420
cctcctggtc tgtgtcagta ttaccaccag actgactcca ggactcactg ccctccagaa 480
aacagaggtg acaaatgtga gggacactgg ggcctttctt ctccttgtag gggtctctca 540
gag                                                               543
```

<210> 18
<211> 515
<212> DNA
<213> Homo sapiens

<400> 18

```
aaaagct gt c  t ct gct t gga  ggt gccct gc  ccat gt t t ga  gct ggt ggag  cagcagcct t  60
cacat ct ggc  ct gccccgac  gt cct gaacc  t gt ccct aga  t t ct t ct gat  gt agagcgac  120
t ggaaagat t  ct t cgact ca  gaagat gaag  act t t gaaat  cct gt ccct t  t gaaaat cct  180
ggggt cgggg  gt ggcacct g  t gagagcct g  gggct cct gg  t gccgct gcg  t t t cat ccat  240
cccgcccgct  cgcct gccga  gggct gcgcc  ccgt gct gcc  t cccccc aga  gggccacccg  300
ct gt gct cgt  ggact gaggc  t gcgct gccc  gggaggcct t  act gct t ggt  gt cagact gc  360
ccagct caga  gt gcccgt ca  gggcct gt gc  at ccgcacgc  ggagccgt ct  gt t aggagct  420
t ccagagcgt  t ct ct cgaca  ct gccagccc  cgt gt t agca  cct gggcct c  agt cccact t  480
gct cccaggc  gccggt t ct g  t ggt t ggt t t  ggaat                              515
```

<210> 19
<211> 392
<212> DNA
<213> Homo sapi ens

<400> 19

```
agcggct t t g  gacgagggca  gaaccacaac  gt gcaagggt  t ccacccct a  ccgacgct ag  60
cccgcggcgc  cgcgacgt ct  gcacggccca  gacccaggat  t ccaaact t g  t gaact cgt g  120
acaat cacaa  act t ggcggc  aaagt ggcga  ct caacct t g  gggggggggg  cgggggg agg  180
gcgcgaggct  t t t ggagcgg  ct gt gggt gt  cgt ct ggact  gaggt t t t t a  aat at t t ct t  240
t ct ct aaccc  at cagcacaa  t aaaaaaaag  t cact ggt t c  aacaacaggg  t t t aaaaaaa  300
at gt ct t cag  ct t t aat t ca  aaact t cagg  t t t ct t t t t c  t t cct t t t t t  t t ggaaat t a  360
t t t t cct gag  cct t t t gt t t  t acggt at at  t g                                     392
```